# EUROPEAN PATENT SPECIFICATION

(11) **EP 2 508 143 B1**
(45) Date of publication and mention of the grant of the patent: **26.02.2014**
(21) Application number: 11795697.9
(22) Date of filing: 13.06.2011
(51) Int. Cl.: A61N 7/00, A61B 19/00, A61B 17/32, A61B 17/00

(54) **ULTRASOUND SUCTION SYSTEM**
ULTRASCHALLSAUGVORRICHTUNG
SYSTÈME D'ASPIRATION D'ULTRASON

(30) Priority: 17.06.2010 US 355646 P
(43) Date of publication of application: 10.10.2012
(73) Proprietor: OLYMPUS MEDICAL SYSTEMS CORP., Tokyo 151-0072 (JP)
(72) Inventor: SAWADA, Yukihiko, Tokyo 151-0072 (JP); YAMADA, Norihiro, Tokyo 151-0072 (JP); TSUKIYAMA, Shusaku, Tokyo 151-0072 (JP)
(74) Representative: Gunzelmann, Rainer
(86) International application number: PCT/JP2011/063511
(87) International publication number: WO 2011/158792

(56) References cited:
- JP-A- 11 155 869
- JP-A- 11 155 869
- JP-A- 2007 296 002
- US-A1- 2010 137 751

## Description

### Technical Field

The present invention relates to an ultrasound suction system that performs suction using ultrasound.

### Background Art

In recent years, various kinds of treatments for medical treatment for patients are widely performed under endoscope observation employing an endoscope.

In order to make it easy to perform treatment, in some cases, an ultrasound suction apparatus or an ultrasound suction system is used that gives ultrasound vibration energy to a treatment target living tissue, fragments a fragile tissue with ultrasound vibration and sucks the fragile tissue, and exposes an abundantly elastic tissue such as a blood vessel without fragmenting the tissue.

In this case, the fragile tissue is emulsified and fluid is supplied to a surface of the treatment target living tissue such that fragmented tissue pieces can be smoothly sucked. Therefore, when the ultrasound vibration energy is given to the treatment target living tissue, since the ultrasound vibration energy is given to the fluid as well, in some cases, the fluid changes to mist and deteriorates an observation function from an observation window of the endoscope.

A gas supply and suction control system disclosed in Japanese Patent Application Laid-Open Publication No. 11-155869 as a first prior example related to the deterioration in the observation function performs, on the basis of a stop signal for output by a high-frequency cauterization apparatus or an ultrasound coagulation dissection apparatus, control for performing suction by sucking means with a delay of a predetermined time together with pressurization because smoke or mist caused by the high-frequency cauterization apparatus or the ultrasound coagulation dissection apparatus prevents observation by an endoscope. According to the control, the smoke or the mist is sucked and removed even after the stop of the output of the high-frequency cauterization apparatus or the like.

Japanese Patent Application Laid-Open Publication No. 2007-296002 as a second prior example discloses contents for supplying pressurized gas to an observation window because smoke or mist caused by a high-frequency cauterization apparatus adheres to the observation window at a distal end of an endoscope and deteriorates an observation field of view, feeding CO2 gas as the pressurized gas along a surface of the observation window, and forming a fluid curtain to prevent the smoke or the mist from reaching the surface of the observation window.

However, the first prior art example cannot appropriately address the amount of generation of mist since this prior art does not monitor the amount of generation of smoke or mist.

In addition, while the second prior art example controls to prevent smoke or mist from reaching the surface of the observation window by forming a fluid curtain, an ultrasound suction system cannot ensure an observation field of view to be in a clear state by means of a fluid curtain.

This leads to a desire for an ultrasound suction system and ultrasound suction method which, when performing a fragmenting processing using ultrasound by supplying a fluid to a surface of a living tissue, can control an ultrasound output or fluid in accordance with the amount of generation of mist to smoothly perform the processing.

The present invention was made in view of the above-mentioned points, and an objective of the present invention is to provide an ultrasound suction system capable of controlling an ultrasound output or fluid in accordance with an amount of generation of mist by means of an observation function of an endoscope and supporting a surgeon to conduct a processing.

### Disclosure of Invention

### Means for Solving the Problem

An ultrasound suction system according to an aspect of the present invention includes the features of claim 1.

### Brief Description of the Drawings

Fig. 1 is a diagram showing, in a used state, an overall configuration of an ultrasound suction system according to a first embodiment of the present invention.
Fig. 2 is a block diagram showing a schematic configuration of the ultrasound suction system.
Fig. 3A is a diagram showing an example of an observed image in a state in which mist does not occur.
Fig. 3B is a diagram showing an example of an observed image in a state in which mist occurs.
Fig. 3C is a diagram showing an example in which mist adheres to an outer surface of an objective lens.
Fig. 4 is a block diagram showing a specific configuration of a mist determining and determination signal generating section.
Fig. 5A is a diagram showing a scanning line serving as a detection region for detecting mist occurrence in an observed image.
Fig. 5B is a diagram showing an example of an image signal of a reference image and an image signal added with a threshold.
Fig. 5C is a diagram showing an example of an image signal of an observed image in the case in which mist occurs.
Fig. 6 is a flowchart for explaining a procedure of an ultrasound suction method in the first embodiment.
Fig. 7 is a timing chart for explaining operation of main parts in Fig. 6.
Fig. 8 is an explanatory diagram of an example in which a maximum value of a reference image is used for a determination reference and a case in which a value obtained by adding a threshold to the maximum value of the reference image is set as the determination reference.
Fig. 9A is a block diagram showing a configuration example of an adhesion determining section in a first modification of the first embodiment.
Fig. 9B is an explanatory diagram in which an image region is divided into plural small regions.
Figs. 10(A) to 10(I) show timing charts for operation explanation of Fig. 9A and Figs. 10(J) to 10(K) are timing charts for operation explanation of a second modification of the first embodiment.
Fig. 11 is a configuration diagram of main parts in a third modification of the first embodiment.
Fig. 12 is a timing chart for operation explanation of the third modification.
Fig. 13A is a perspective view showing a configuration of a modification of the third modification.
Fig. 13B is a perspective view showing a configuration of another modification of the third modification.
Fig. 14A is a side view showing a schematic configuration of an ultrasound suction probe in a second embodiment of the present invention.
Fig. 14B is a side view showing a schematic configuration of the ultrasound suction probe in the case in which an operation lever is operated in Fig. 14A.
Fig. 14C is a perspective view showing a configuration of an umbrella with an external sheath removed;
Fig. 14D is a perspective view showing a configuration of the umbrella in the case in which the operation lever is operated in a state of Fig. 14C.
Fig. 15 is a diagram showing a state in which treatment is performed in the second embodiment.
Fig. 16A is a side view showing a configuration on a distal end side of an ultrasound suction probe in a third embodiment of the present invention.
Fig. 16B is a perspective view showing a configuration of a distal end side of the ultrasound suction probe in the third embodiment.
Fig. 17 is a diagram showing a state in which treatment is performed in the third embodiment.
Fig. 18 is a block diagram showing a configuration of a part of an ultrasound suction system in a fourth embodiment.
Figs. 19(A) to 19(I) are timing charts for operation explanation of the fourth embodiment.

### Best Mode for Carrying Out the Invention

Embodiments of the present invention are explained below with reference to the drawings.

### (First Embodiment)

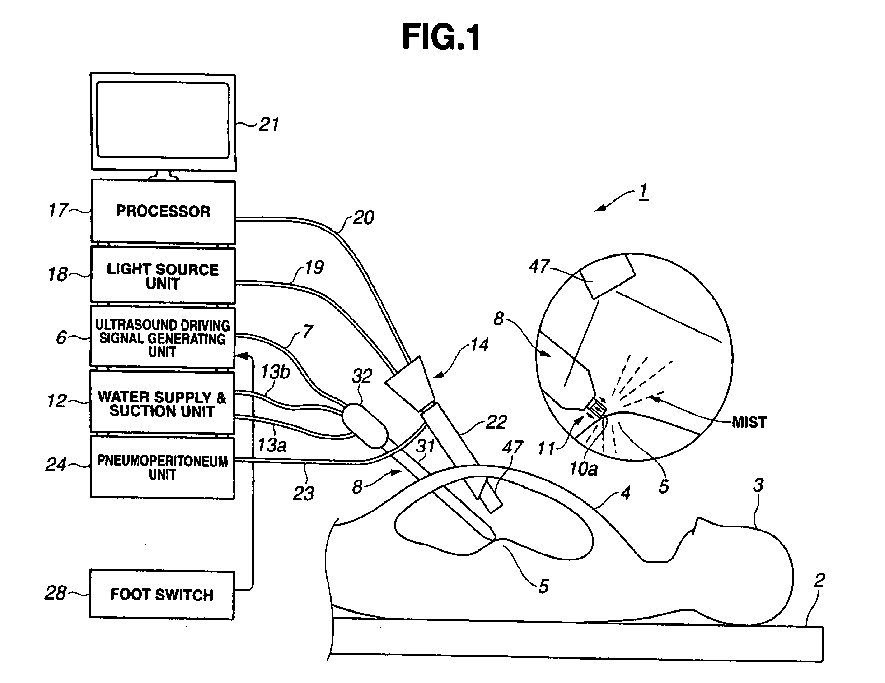

As shown in Fig. 1, an ultrasound suction system 1 according to a first embodiment of the present invention applies, using ultrasound vibration energy (abbreviated as ultrasound vibration), treatment to, for example, a living tissue of a diseased part 5 set as a treatment target in an abdomen 4 of a patient 3 lying on a bed 2.

Therefore, the ultrasound suction system 1 includes an ultrasound driving signal generating unit 6 that generates an ultrasound driving signal for generating ultrasound vibration. The ultrasound driving signal generating unit 6 outputs the generated ultrasound driving signal to an ultrasound suction probe 8 functioning as an ultrasound suction section via a signal cable 7.

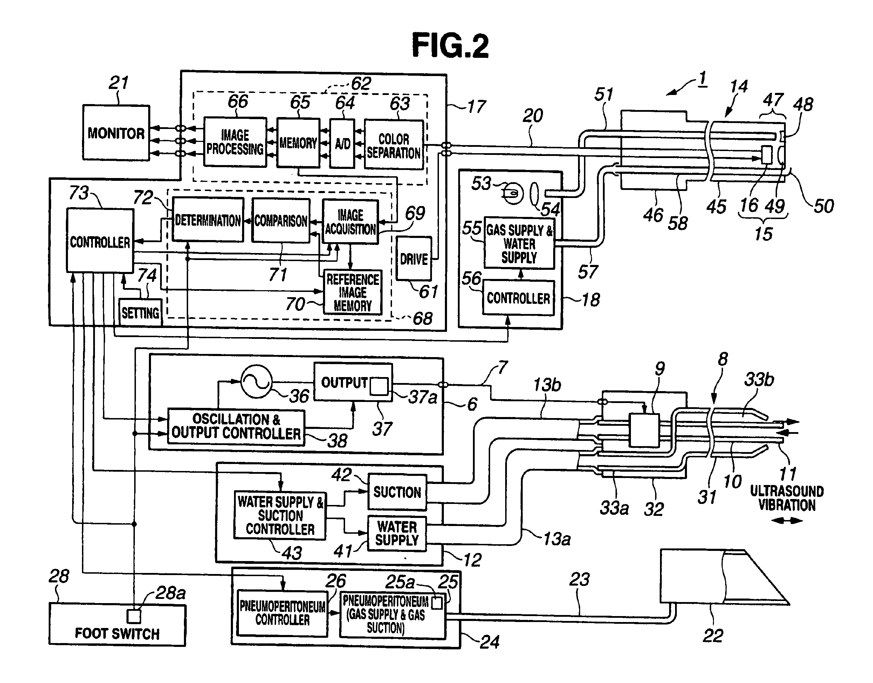

As shown in Fig. 2, the ultrasound suction probe 8 incorporates an ultrasound transducer 9 functioning as an ultrasound generating section that generates ultrasound vibration by being applied with the ultrasound driving signal from the ultrasound driving signal generating unit 6 via the signal cable 7. With the ultrasound transducer 9, the generated ultrasound vibration is transmitted to a distal end portion 11 of the ultrasound suction probe 8 via a transmission pipe 10 functioning as a vibration transmitting section that transmits the ultrasound vibration.

A surgeon brings the distal end portion 11 into contact with a surface of the living tissue of the diseased part 5, whereby the ultrasound vibration is given to the living tissue of the diseased part 5 and a fragile living tissue portion such as a fat tissue in the living tissue is fragmented. Further, fragmented living tissue pieces are emulsified under presence of fluid (specifically, a physiological salt solution) supplied to a living tissue surface explained below.

The ultrasound suction system 1 includes a water supply and suction unit 12 included in a fluid supplying section such that the fragmented living tissue pieces can be smoothly removed by suction. A water supply device 41 in the water supply and suction unit 12 supplies the physiological salt solution (simply abbreviated as water) serving as fluid via a water supply tube 13a provided in the ultrasound suction probe 8. A suction device 42 sucks the fragmented living tissue pieces as emulsion and the water via a suction tube 13b provided in the ultrasound suction probe 8.

The ultrasound suction system 1 includes an endoscope 14 that optically observes the diseased part 5, the distal end side of the ultrasound suction probe 8, and the like, a processor 17 that performs signal processing for an image pickup device 16 included in an image pickup section 15 (shown in Fig. 2) functioning as an observing section provided in the endoscope 14, and a light source unit 18 for illuminating an image pickup range in which an image is picked up by the image pickup section 15.

A light guide cable 19 extended from the endoscope 14 is connected to the light source unit 18. A signal cable 20 extended from the endoscope 14 is connected to the processor 17.

An image signal (a video signal) generated by image processing by the processor 17 is outputted to a monitor 21 functioning as image display means. An image picked up by the image pickup section 15 is displayed on a display surface of the monitor 21 as an endoscope image.

As shown in Fig. 1, the endoscope 14 is inserted into the abdomen 4 via a trocar 22. The ultrasound suction probe 8 is also inserted into the abdomen 4 actually via a trocar. As shown in Fig. 2, a pneumoperitoneum tube 23 connected to the trocar 22 is connected to a pneumoperitoneum unit 24.

The pneumoperitoneum unit 24 supplies gas for pneumoperitoneum into the abdomen 4 via the pneumoperitoneum tube 23 by a gas supply and gas suction device included in a pneumoperitoneum device 25, inflates an inside of the abdomen 4 with the gas, changes the inside of the abdomen to a state in which observation and treatment can be easily performed. The pneumoperitoneum device 25 can also suck the gas in the abdomen 4 via the pneumoperitoneum tube 23. By sucking the gas, the pneumoperitoneum device 25 can quickly set the inside of the abdomen 4 to predetermined pressure.

Operation of gas supply and gas suction of the pneumoperitoneum device 25 is controlled by a pneumoperitoneum controller 26. The pneumoperitoneum device 25 includes a pressure sensor 25a such that pressure control can be performed by the pneumoperitoneum controller 26. Atmospheric pressure in the abdomen 4 can be kept constant according to pressure information by the pressure sensor 25a.

In the ultrasound suction system 1, a foot switch 28 is provided as a user interface with which a user such as a surgeon applies instruction operation to the ultrasound driving signal generating unit 6, the water supply and suction unit 12, the light source unit 18, the pneumoperitoneum unit 24, and the like.

In the foot switch 28, an ultrasound switch 28a as an instruction operation switch for applying instruction operation for generation (ON) and stop (OFF) of an ultrasound driving signal to the ultrasound driving signal generating unit 6 is provided.

In the foot switch 28, in order to spout fluid from a nozzle 50 functioning as a fluid spouting section explained later, a gas supply and water supply switch for applying instruction operation for ON/OFF of gas supply and water supply to the gas supply and water supply unit 55, a pneumoperitoneum switch for performing instruction operation for ON/OFF of pneumoperitoneum (gas supply and gas suction) of the pneumoperitoneum unit 24, and the like may be provided.

Fig. 2 shows main components of the units in Fig. 1. The ultrasound suction probe 8 includes a slender outer pipe (or sheath) 31 and the transmission pipe 10 that is coaxially inserted through the outer pipe 31 and transmits ultrasound. A gripping portion 32 expanded in diameter is provided on a proximal end side in the outer pipe 31. The ultrasound transducer 9 is arranged on an inside of the gripping portion 32.

The ultrasound transducer 9 is provided in a ring shape, for example, near a rear end of the transmission pipe 10. Ultrasound vibration by the ultrasound transducer 9 is transmitted to the distal end portion 11 by the transmission pipe 10. As indicated by an arrow in Fig. 2, the distal end portion 11 ultrasonically vibrates in an axis direction thereof.

An internal space of the transmission pipe 10 forms a suction conduit. A rear end of the transmission pipe 10 is connected to the water supply and suction unit 12 via the suction tube 13b.

On the other hand, the water supply tube 13a is connected to a first water supply pipe 33a provided on a proximal end side of the ultrasound suction probe 8. The first water supply pipe 33a communicates with a second water supply pipe 33b between the transmission pipe 10 and the outer pipe 31 halfway in the first water supply pipe 33a. The distal end of the outer pipe 31 has a tapered-off shape and opens slightly behind the distal end of the transmission pipe 10. As indicated by an arrow, water is supplied from a ring-like opening portion on the outer side of the transmission pipe 10.

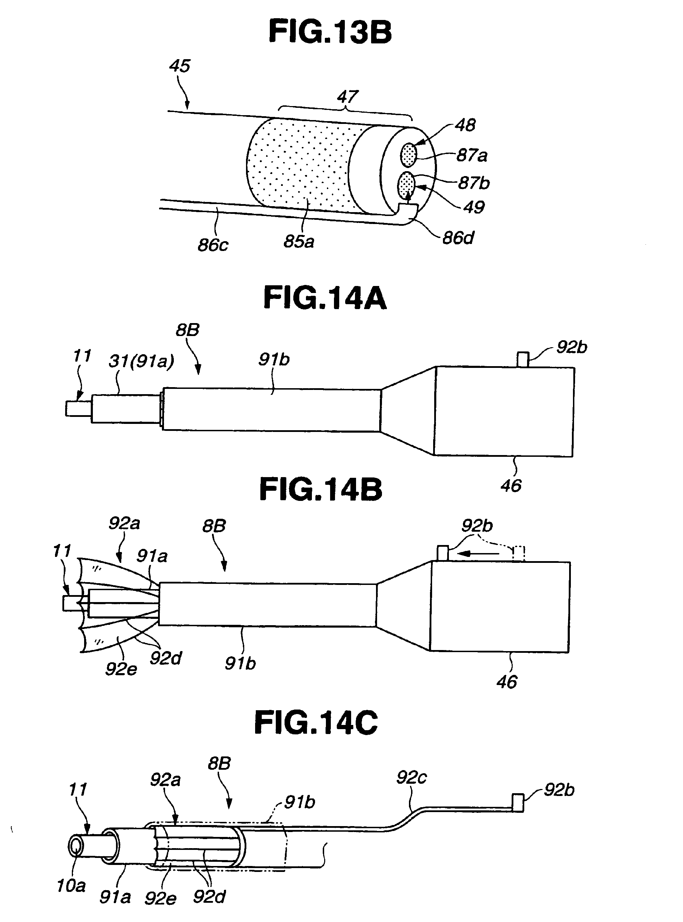

In Fig. 2, the distal end of the outer pipe 31 has the tapered-off shape but is not limited to the tapered-off shape. For example, in Fig. 14A, the distal end side of the outer pipe 31 has a circular tube shape.

On the other hand, the distal end opening of the transmission pipe 10 is a suction port 10a. Fragmented living tissue pieces and the like are sucked from the suction port 10a while being mixed in the water supplied as indicated by the arrow. In Fig. 1, in a circular enlarged view, a state in which treatment is applied to a living tissue is shown.

The ultrasound driving signal generating unit 6 includes an oscillator 36 that generates an AC oscillation signal, an output circuit 37 that amplifies the oscillation signal and insulates the oscillation signal and outputs the oscillation signal as an ultrasound driving signal, and an oscillation and output controller 38 that performs control of oscillation and oscillation stop by the oscillator 36 and control of output from the output circuit 37.

The ultrasound transducer 9 of the ultrasound suction probe 8 is applied with an ultrasound driving signal from the output circuit 37 via the signal cable 7. The ultrasound transducer 9 generates ultrasound or generates ultrasound vibration. The output circuit 37 incorporates an amplifier or attenuator 37a that changes a current value of the ultrasound driving signal outputted to the ultrasound transducer 9. An output controller of the oscillation and output controller 38 controls the amplifier or attenuator 37a to perform control including a reduction in the output of the ultrasound driving signal. When the output of the ultrasound driving signal is changed, the output controller may perform the control including a reduction in the output of the ultrasound driving signal by changing amplitude of the ultrasound driving signal.

The foot switch 28 is connected to the oscillation and output controller 38. The surgeon can operate the foot switch 28 and perform an output instruction, an output stop (oscillation stop) instruction, and the like for the ultrasound driving signal.

The water supply and suction unit 12 includes the water supply device 41 that performs water supply and the suction device 42 that performs suction as explained above and a water supply and suction controller 43 that controls operation of the water supply device 41 and the suction device 42.

The water supply device 41 is connected to the water supply tube 13a and supplies water to the water supply pipes 33a and 33b side of the ultrasound suction probe 8 via the water supply tube 13a.

The suction device 42 is connected to the suction tube 13b and sucks water from the suction port 10a at the distal end of the transmission pipe 10 via the transmission pipe 10 having a function of a suction conduit of the ultrasound suction probe 8 connected to the suction tube 13b.

The endoscope 14 includes an elongated insertion portion 45 and a gripping portion 46 provided at a rear end of the insertion portion 45. An illuminating window and an observation window are provided at a distal end portion 47 of the insertion portion 45. An illumination lens 48 is attached to the illuminating window to form an illumination section. An objective lens 49 is attached to the observation window. The image pickup device 16 is arranged at an image-forming position of the objective lens 49 to form the image pickup section 15 functioning as the observing section.

Near the objective lens 49 that forms an observation field of view by the observing section, the nozzle 50, a spout port of which faces the objective lens 49, is arranged.

Illuminating light from the light source unit 18 is transmitted by a light guide 51 inserted through the endoscope 14 and emitted from an end of the light guide 51 via the illumination lens 48. An optical image of the diseased part 5 or the like illuminated by the illuminating light from the illumination lens 48 is formed by the objective lens 49 on the image pickup device 16 arranged at an image-forming position of the objective lens 49.

The light source unit 18 collects, with a lens 54, illuminating light generated by a light source lamp 53 and makes the illuminating light incident on a proximal end of the light guide 51. The light source unit 18 includes a gas supply and water supply unit 55 that supplies gas and water. Operation by the gas supply and water supply unit 55 is controlled by a gas supply and water supply controller (abbreviated as controller) 56.

The gas supply and water supply unit 55 is connected to a gas supply and water supply tube 58, which is provided in the endoscope 14, via a gas supply and water supply tube 57. The gas supply and water supply unit 55 spouts gas and water from the nozzle 50 included in the fluid spouting section provided at a distal end of the gas supply and water supply tube 58 to an outer surface of the objective lens 49 of the observation window. According to the spouting operation, deposit adhering to the outer surface is removed to make it possible to keep the observation window in a clean state, i.e., secure a satisfactory observation field of view. The fluid spouting section may be defined to include the nozzle 50 and the gas supply and water supply unit 55.

The processor 17 to which the signal cable 20 is connected includes an image pickup device drive circuit (abbreviated as drive circuit) 61 connected to the image pickup device 16. The drive circuit 61 applies the image pickup device with a drive signal. The image pickup device 16 outputs, by being applied with the drive signal, an image signal obtained by photoelectrically converting an optical image formed on an image pickup surface.

The image signal outputted from the image pickup device 16 is color-separated into image signals of color components by a color separation circuit 63 included in an image processing unit 62 in the processor 17 and then converted into digital image signals of the color components by an A/D converter 64 and temporarily recorded (stored) in a memory 65.

The image signals of the color components recorded by the memory 65 are further subjected to image processing such as gamma correction by the image processing circuit 66. A standard image signal is generated and outputted to the monitor 21. The surgeon performs treatment by the ultrasound suction probe 8 while observing, as an observed image, an endoscope image displayed on the monitor 21.

As explained above, the surgeon can fragment a fragile living tissue and efficiently suck water mixed with fragmented living tissue pieces (emulsion) with the ultrasound suction probe 8 while performing water supply and suction. However, in some cases, because of ultrasound vibration in a state in which water is present, a phenomenon in which water or emulsion in which water and living tissue pieces are mixed scatters as mist occurs.

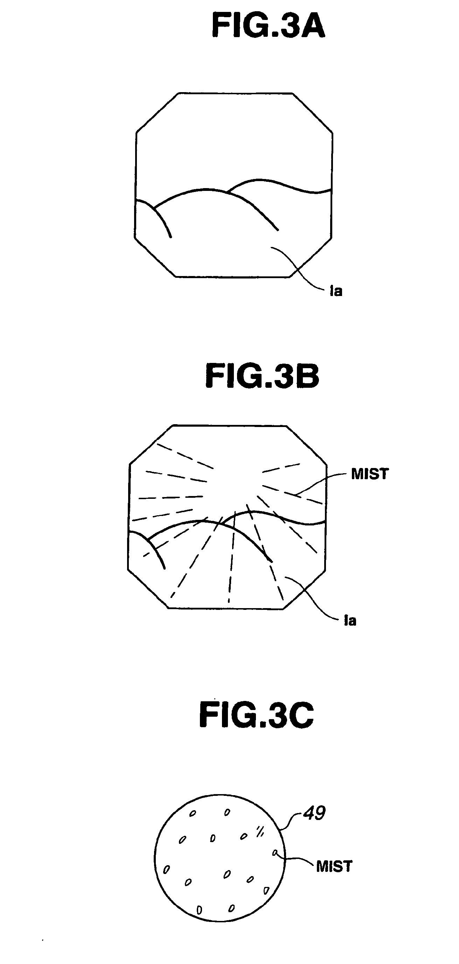

In a state in which mist hardly occurs or a state in which ultrasound vibration is stopped, for example, as shown in Fig. 3A, an observed image in which an image of a living tissue portion indicated by a sign Ia can be satisfactorily observed is obtained. In a state of the observed image Ia shown in Fig. 3A, when mist exceeding a predetermined value occurs, the observed image changes to an observed image involving linear mist having high luminance such as fireworks or rain in the observed image Ia as schematically shown in Fig. 3B.

In such an observed image involving the line-like mist, it is difficult for the surgeon to observe the observed image Ia in the treatment target living tissue portion in a satisfactory observation state shown in Fig. 3A. In such a case, the surgeon turns off the ultrasound switch 28a to stop the ultrasound vibration and suspend treatment by the ultrasound vibration. The surgeon stops the occurrence of mist or reduces the occurrence of mist to a predetermined value or less and checks a state of the living tissue and then turns on the ultrasound switch 28a again. Thereafter, work for suspending the treatment in the same manner when mist exceeding the predetermined value occurs is repeated.

When such mist exceeding the predetermined value frequently occurs, scattering mist adheres to the outer surface of the objective lens 49 as deposit. When the mist scatters and adheres to the outer surface of the objective lens 49, the mist adheres, for example, as shown in Fig. 3C. When the mist adheres to the outer surface of the observation window, i.e., the outer surface of the objective lens 49 as shown in Fig. 3C, the observed image shown in Fig. 3A changes to an unclear observed image because of the adhering mist.

Therefore, in this embodiment, an observed image in a state in which mist does not (substantially) occurs is acquired and a reference image serving as a reference is set in advance as explained below. A following observed image is compared with the reference observed image. When a change equal to or larger than a predetermined value is detected, it is determined that the observed image is an observed image in which mist exceeding the predetermined value occurs. A mist determination signal corresponding to the occurrence of the mist exceeding the predetermined value is generated from a result of the determination.

As shown in Fig. 2, the image signals of the color components recorded in the memory 65 in the processor 17 are inputted to an image acquisition circuit 69 included in a mist determining and determination signal generating section 68. In this case, the image acquisition circuit 69 may acquire an image signal of one color component or may acquire plural image signals. A luminance signal Y may be generated from, for example, color signals of R, G, and B. The image acquisition circuit 69 may acquire the luminance signal Y.

The image acquisition circuit 69 generates (an image signal of) a reference image for detecting occurrence of mist exceeding the predetermined value from the acquired image signals and acquires, in time series, observed images for which it is determined whether mist exceeding the predetermined value occurs.

The image acquisition circuit 69 extracts, for example, an image portion of a predetermined region from an observed image picked up at the time (timing) when the ultrasound switch 28a is changed from OFF to ON from a state in which mist does not occur and records the image portion in a reference image memory 70 as a reference image.

As timing for recording (acquiring) the reference image as explained below, besides the time when the ultrasound switch 28a is turned on, arbitrary timing up to time when the ultrasound switch 28a is turned on or immediately after the ultrasound switch 28a is turned on may be set.

A state up to the time when the ultrasound switch 28a is turned on or immediately after the ultrasound switch 28a is turned on is equivalent to a state at a time when the ultrasound transducer 9 is applied with an ultrasound driving signal, the ultrasound transducer 9 ultrasonically vibrates, and the ultrasound vibration is given to the distal end portion 11 of the ultrasound suction probe 8 via the transmission pipe 10 or immediately after the ultrasound vibration is given. An observed image (or an image portion of a predetermined region of the observed image) picked up in a state in which mist substantially does not occur from the time when the ultrasound vibration is given until immediately after the ultrasound vibration is given may be set as a reference image. In the following explanation, a case of the timing when the ultrasound switch 28a is turned on equivalent to the time when the ultrasound vibration is given is mainly explained.

In a state in which the ultrasound switch 28a after the acquisition of the reference image is turned on, the image acquisition circuit 69 extracts a part of an observed image of the same predetermined region from the memory 65 and outputs the part of the observed image to a comparison circuit 71.

The comparison circuit 71 compares the reference image recorded in the reference image memory 70 and the observed image acquired by the image acquisition circuit 69 in the state in which the ultrasound switch 28a is turned on.

The comparison circuit 71 outputs a comparison result to a determination circuit 72. The determination circuit 72 determines, from the comparison result by the comparison circuit 71, whether there is a change equal to or larger than a predetermined value and outputs a determination result to a controller 73 included in a control section. Only when the determination circuit 72 determines that there is a change equal to or larger than the predetermined value in the observed image in comparison with the reference image, the determination circuit 72 determines that mist exceeding the predetermined value occurs and outputs a mist determination signal.

The controller 73 has a function of the control section for controlling, according to a determination result by the determination circuit 72, at least operation of the ultrasound driving signal generating unit 6 and the water supply and suction unit 12. When the mist determination signal is inputted, the controller 73 performs control for causing the ultrasound driving signal generating unit 6 to stop or reduce the output of the ultrasound driving signal and performs control for causing the water supply and suction unit 12 to stop operation of water supply and suction or reduce an operation function (of water supply and suction).

The controller 73 can control the sections in the processor 17 and, by sending control signals to the controllers 38, 43, 26, and 56 of the ultrasound driving signal generating unit 6, the water supply and suction unit 12, the pneumoperitoneum unit 24, the light source unit 18 included in the ultrasound suction system 1, can control operations of the units.

The operation of the ultrasound driving signal generating unit 6, the water supply and suction unit 12, the pneumoperitoneum unit 24, and the light source unit 18 may be concentratedly controlled directly according to the control signals by the controller 73 not via the controllers 38, 43, 26, and 56.

In the processor 17, a setting section 74 is provided that sets a reference image and performs, besides determination of occurrence of mist exceeding the predetermined value, setting operation for an operation mode for determining whether deposit due to scattering of mist adheres to the outer surface of the object lens 49 and the objective lens 49 changes to a state of an unclear observation field of view as explained later. The user such as the surgeon can perform setting and selection of a reference image, selection of an operation mode, and the like from the setting section 74.

A signal in the case of the setting and selection from the setting section 74 is inputted to the controller 73. The controller 73 performs setting and selection of a reference image and control of an operation mode according to the signal.

An output mode setting section may be provided that selects and sets a continuous output mode for continuously outputting an ultrasound driving signal and an intermittent output mode for intermittently outputting an ultrasound driving signal when the ultrasound switch 28a is turned on. The ultrasound driving signal generating unit 6 may be caused to operate in the intermittent output mode as shown in a figure in a fourth embodiment explained later.

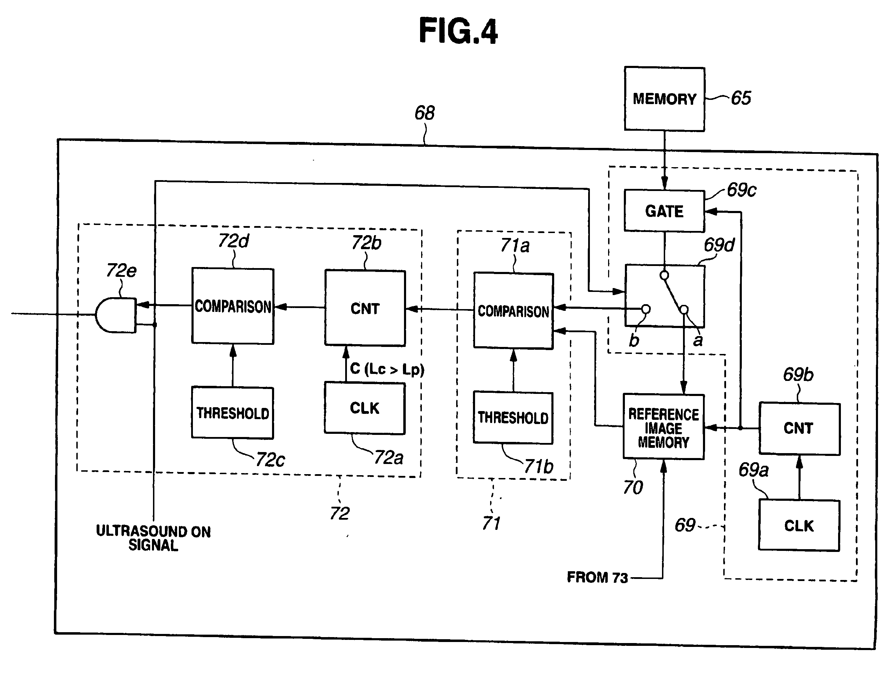

Fig. 4 shows a more specific configuration example of the mist determining and determination signal generating section 68.

Image signals of frames are read out from the memory 65 according to an address signal based on a clock from a clock circuit 69a included in the image acquisition circuit 69, outputted to the image processing circuit 66, and inputted to a gate 69c via a counter 69b.

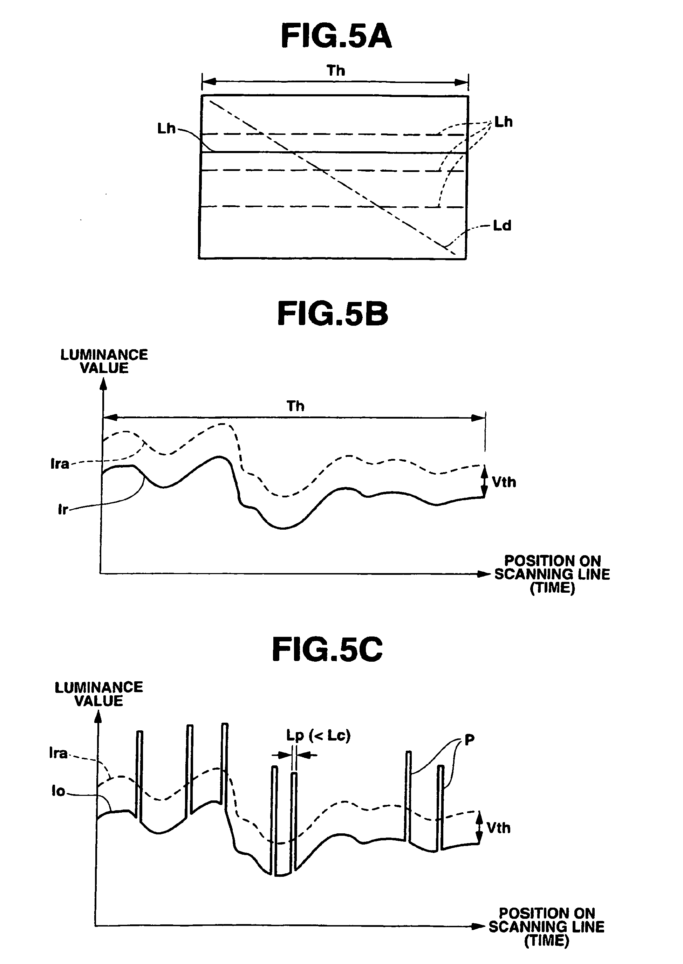

The gate 69c is open in a period corresponding to a predetermined region corresponding to a setting value set by the counter 69b. An image signal in that period is stored in the reference image memory 70 as a reference image via a switch 69d. A solid line in Fig. 5A indicates a scanning line Lh serving as the predetermined region. The image signal passes through the gate 69c in a period Th corresponding to the scanning line Lh.

The predetermined region corresponding to one scanning line Lh is indicated by the solid line in Fig. 5A. However, the predetermined region may be set by plural scanning lines Lh as indicated by dotted lines.

The predetermined region is not limited to the scanning line Lh in the horizontal direction. As indicated by an alternate long and two short dashes line, for example, the predetermined region for performing mist determination may be set in an image portion on a diagonal line Ld. Besides, the predetermined region may be set along the vertical direction.

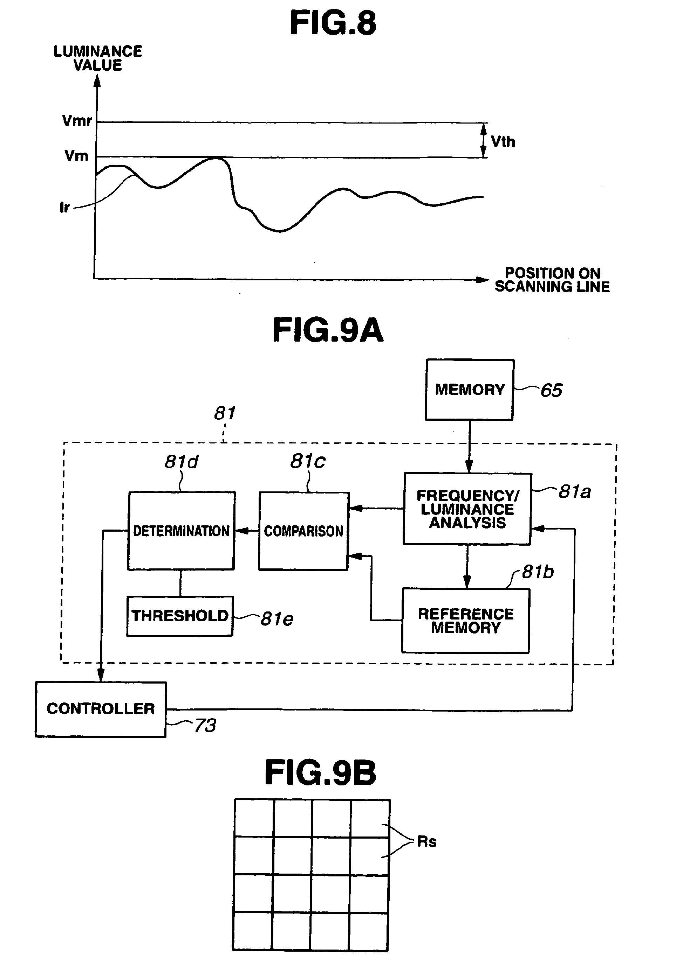

For example, as shown in Fig. 9B, an image region may be divided into plural small regions Rs as shown in Fig. 9B. One or plural small regions Rs may be set as the predetermined region for performing mist determination. A predetermined period may be set instead of the predetermined region.

In the switch 69d, a contact a is selected in a state in which an ultrasound ON signal by ON operation of the ultrasound switch 28a is not inputted. A contact b is selected when the ultrasound ON signal is inputted. Therefore, in a state in which the contact a is selected, the reference image of the reference image memory 70 is sequentially updated to new reference images. When the ultrasound ON signal is generated, a part of an observed image acquired by the observing section at timing of the ultrasound ON signal is frozen in the reference image memory 70 as a reference image.

When the contact b is selected, the image signal from the memory 65 is inputted to the comparison circuit 71 in a predetermined horizontal period when the gate 69c is open. In this case, the image signal of the reference image recorded in the reference image memory 70 is also inputted to the comparison circuit 71 in synchronization with the open of the gate 69c.

The comparison circuit 71 compares two image signals each inputted in synchronization with the predetermined period Th. The comparison circuit 71 performs comparison to determine whether an image signal of an observed image has a sufficiently high luminance value compared with a luminance value of the reference image rather than directly comparing luminance values of both the image signals.

In order to perform such comparison, the comparison circuit 71 includes a comparator 71 a and a threshold setting device 71b (in Fig. 4, simply abbreviated as threshold 71b stored therein). The same holds true concerning a threshold setting device 72c explained later.

The comparator 71a compares a luminance value obtained by adding the threshold 71b (set by the threshold setting device 71b) to an image signal Ir of the reference image and an image signal of an observed image and outputs a comparison result to the determination circuit 72.

In Fig. 5B, an image signal Ira of the reference image set by adding a threshold Vth to the image signal of the reference image indicated by the solid line is indicated by a dotted line. In a state in which mist does not occur, a luminance value of the image signal substantially continuously changes. A value of the change is not so large in a short period.

On the other hand, an image signal in a state in which mist exceeding the predetermined value occurs is an image signal Io involving pulses P in which the luminance value sharply changes in a pulse (or line) shape (because illuminating light is reflected by the mist) as shown in Fig. 5C. When the mist exceeding the predetermined value occurs, it frequently occurs that plural pulses P are discretely involved, for example, on the scanning line Lh serving as the predetermined region.

By comparing the luminance value of the image signal Ira shown in Fig. 5B and the luminance value of the image signal Io shown in Fig. 5C, it is possible to effectively determine the occurrence of the mist exceeding the predetermined value.

The determination circuit 72 counts, with a counter 72b, an output signal of the comparison circuit 71 in synchronization with a predetermined clock by a clock circuit 72a having a predetermined period. As explained above, since the mist occurs to increase the luminance value in a pulse shape, the determination circuit 72 counts, with the counter 72b, the output value using a clock C corresponding to width La slightly larger than average pulse width Lp of the luminance value (La>Lp).

The determination circuit 72 determines, with a comparison circuit 72d, whether a count value of the counter 72b is larger than a threshold 72c. When the count value of the counter 72b is larger than the threshold 72c, the comparison circuit 72d outputs a mist determination signal to the controller 73 via an AND circuit 72e. In this way, it is possible to effectively determine a pulse-like image involving the pulses P, which is a characteristic of the mist, according to image processing based on an observed image.

For example, the ultrasound ON signal from the ultrasound switch 28a of the foot switch 28 is inputted to the AND circuit 72e. Only in a state in which the ultrasound ON signal is inputted, the determination circuit 72 generates the mist determination signal indicating determination of the occurrence of the mist exceeding the predetermined value.

When the mist determination signal is inputted, the controller 73 generates a suppression signal for suppressing the occurrence of the mist. The controller 73 performs, according to the suppression signal, control for stopping or reducing generation of an ultrasound driving signal via the oscillation and output controller 38 of the ultrasound driving signal generating unit 6.

The controller 73 performs, according to the suppression signal, control for stopping or reducing water supply of the water supply device 41 via the controller 43 of the water supply and suction unit 12. The generation of the ultrasound driving signal is stopped, whereby ultrasound vibration by the ultrasound transducer 9 is quickly stopped. Therefore, when the mist exceeding the predetermined value occurs, the occurrence of the mist is quickly reduced or stopped according to the stop of the ultrasound vibration.

On the other hand, even if the water supply of the water supply device 41 is stopped, a temporal delay occurs in actually stopping water supply from a vicinity of the distal end portion 11 of the ultrasound suction probe 8. Therefore, when the occurrence of the mist exceeding the predetermined value is reduced or stopped with high responsiveness and in a short period, only the generation of the ultrasound driving signal may be stopped without stopping the water supply. The operation of the water supply and the operation of the suction are more desirably associated with each other (i.e., when supply of water as fluid is reduced or stopped, suction is associated with the reduction or the stop of the water supply to be reduced or stopped). However, in a short period, only the operation of the water supply may be stopped.

Instead of completely stopping the generation of the ultrasound driving signal, an output value of the ultrasound driving signal may be controlled to be reduced. Instead of stopping the operation of the water supply, a water supply amount may be controlled to be reduced. Instead of stopping the operation of the suction, a suction amount may be controlled to be reduced.

The ultrasound suction system 1 having the configuration explained above includes the ultrasound driving signal generating unit 6 functioning as an ultrasound driving signal generating section that generates an ultrasound driving signal and the ultrasound suction probe 8 in which the ultrasound transducer 9 functioning as the ultrasound generating section that generates ultrasound vibration energy according to application of the ultrasound driving signal and the suction port 10a for transmitting the ultrasound vibration energy generated by the ultrasound generating section to the distal end portion 11 and giving the ultrasound vibration energy to a treatment target living tissue from the distal end portion 11, fragmenting the living tissue, and sucking fragmented living tissue pieces are provided.

The ultrasound suction system 1 includes the endoscope 14 in which the image pickup section 15 functioning as the observing section that optically observes the living tissue is provided at the distal end portion 47 of the insertion portion 45, the water supply device 41 included in the fluid supplying section that supplies fluid to a surface of the living tissue, and the image acquisition circuit 69 functioning as the image acquiring section for acquiring, via the observing section, an observed image in which mist is likely to occur from the surface of the living tissue in a state in which the ultrasound vibration energy is given. The ultrasound suction system 1 includes the controller 73 functioning as the control section that controls, on the basis of a comparison result obtained by comparing the reference image corresponding to a state in which the mist does not (substantially) occur and the observed image acquired by the image acquiring section, output of the ultrasound driving signal by the ultrasound driving signal generating section.

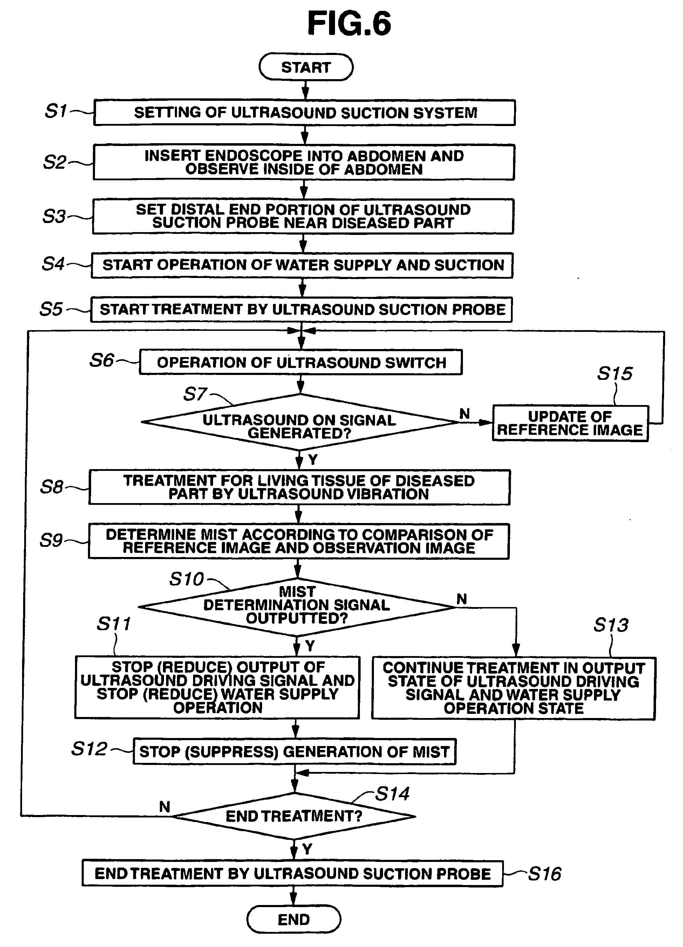

With reference to Fig. 6, a procedure of an ultrasound suction method in performing treatment for a living tissue of the diseased part 5 using the ultrasound suction probe 8 according to this embodiment is explained.

In first step S1, the surgeon sets the ultrasound suction system 1 in a treating state as shown in Fig. 1. The surgeon connects the ultrasound suction probe 8 to the ultrasound driving signal generating unit 6 and the water supply and suction unit 12.

In next step S2, the surgeon inserts the insertion portion 45 of the endoscope 14 into the abdomen 4 of the patient 3 via the trocar 22 to enable the diseased part 5 or the like in the abdomen 4 to be observed by the observing section of the endoscope 14.

In next step S3, under observation by the endoscope 14, the surgeon inserts the ultrasound suction probe 8 into the abdomen 4 and sets the distal end portion side of the ultrasound suction probe 8 near the living tissue of the diseased part 5 to be opposed to the living tissue.

In next step S4, the surgeon sets the water supply and suction unit 12 in an operation state for water supply and suction, i.e., a state in which water is perfused. The ultrasound driving signal generating unit 6 changes to a state in which the ultrasound driving signal generating unit 6 generates an ultrasound driving signal only when the ultrasound switch 28a in the foot switch 28 is turned on.

In next step S5, under observation by the endoscope 14, the surgeon operates the ultrasound switch 28a and starts treatment by the ultrasound suction probe 8 for the living tissue of the diseased part 5.

As shown in steps S6 and S7, the surgeon performs operation for stepping on the ultrasound switch 28a and stops operation for stepping on the ultrasound switch 28a. When the surgeon performs the operation for stepping on the ultrasound switch 28a, the ultrasound switch 28a generates an ultrasound ON signal. When the surgeon stops the operation for stepping on the ultrasound switch 28a, the ultrasound ON signal is stopped being generated (turned off).

When the ultrasound ON signal is generated, as shown in step S8, the ultrasound transducer 9 ultrasonically vibrates. Under observation by the endoscope 14, the surgeon can perform treatment by the ultrasound vibration by bringing the distal end portion of the ultrasound suction probe 8 into contact with the living tissue of the diseased part 5.

When the ultrasound ON signal is generated, as shown in step S9, the mist determining and determination signal generating section 68 freezes a reference image in the reference image memory 70 at timing of the ultrasound ON signal and decides the reference image. The mist determining and determination signal generating section 68 acquires an observed image after the ultrasound ON signal, compares the observed image with the reference image, and starts determination of occurrence of mist exceeding the predetermined value.

In next step S10, the determining section of the mist determining and determination signal generating section 68 determines presence or absence of occurrence of mist exceeding the predetermined value and outputs a determination result. When a mist determination signal is outputted, as shown in step S11, the controller 73 performs control for stopping (or reducing) output of the ultrasound driving signal for a short time and stopping (or reducing) water supply operation for a short time.

When mist exceeding the predetermined value occurs as shown in step S12, the occurrence of the mist can be stopped or suppressed by stopping the output of the ultrasound driving signal. By setting a state in which mist does not occur or is suppressed, the surgeon can perform satisfactory observation using an observed image in which occurrence of mist is stopped or reduced.

On the other hand, when the mist determination signal is not outputted, the control shown in step S11 is not performed. Specifically, when the mist determination signal is not outputted, as shown in step S13, the surgeon continues the treatment by the ultrasound suction probe 8 in an output state of the ultrasound driving signal and a water supply operation state while looking at the observed image.

After processing in step S11 or S13, when instruction operation for ending treatment shown in step S14 is not performed, the processing returns to step S6. The processing in step S6 and subsequent steps explained above is repeated.

When the ultrasound ON signal is not generated in step S7, as shown in step S15, the mist determining and determination signal generating section 68 performs processing for updating the reference image. After the processing, the processing returns to the processing in step S6.

When the treatment for the living tissue of the diseased part 5 ends in this way, as shown in step S16, the treatment by the ultrasound suction probe 8 is ended.

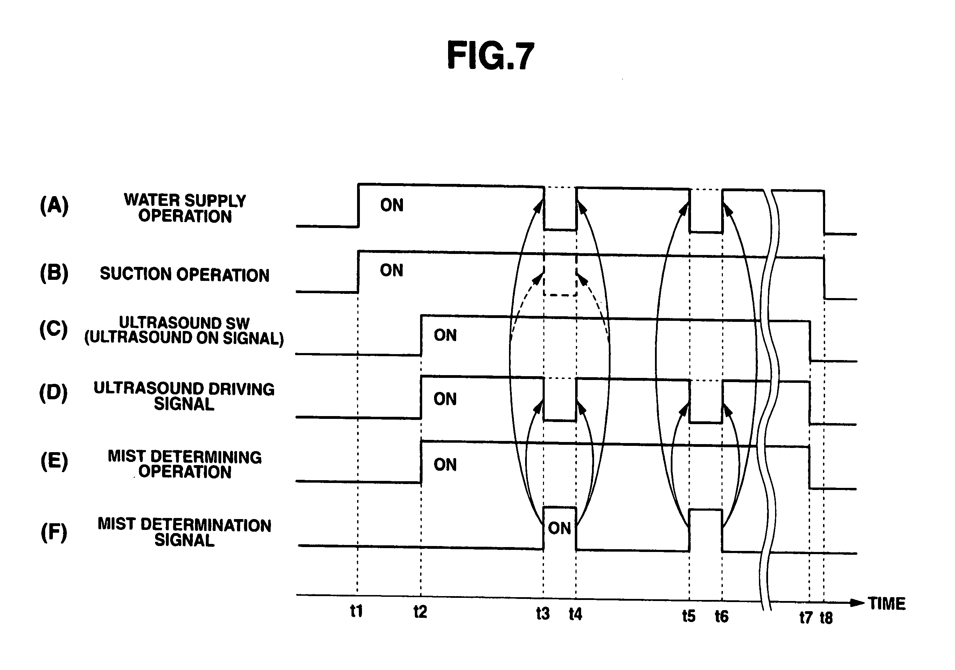

Figs. 7(A) to 7(F) show timing charts for explaining operation of main parts in Fig. 6. According to step S4 of Fig. 6, as shown in Fig. 7(A) and Fig. 7(B), operation of water supply and suction is started, for example, at time t1. As shown in Fig. 7(C), the ultrasound switch 28a is turned on at time t2 after the time t1. When the ultrasound switch 28a is turned on, an ultrasound ON signal is generated. As shown in Fig. 7(D), the ultrasound driving signal generating unit 6 outputs an ultrasound driving signal to the ultrasound transducer 9. The ultrasound transducer 9 ultrasonically vibrates. As shown in step S8 of Fig. 6, the surgeon performs treatment using the ultrasound suction probe 8.

When the ultrasound switch 28a is turned on, as shown in Fig. 7(E), the mist determining and determination signal generating section 68 starts a mist determination operation for determining presence or absence of occurrence of mist. The determination circuit 72 of the mist determining and determination signal generating section 68 outputs a determination result.

As shown in the enlarged view of Fig. 1, it is possible to fragment a fragile fat tissue or the like and expose an abundantly elastic blood vessel or the like by bringing the distal end portion 11 of the ultrasound suction probe 8 into contact with the surface of the living tissue of the diseased part 5 to give ultrasound vibration to the living tissue.

By supplying water and performing suction near the distal end portion 11, it is possible to efficiently suck fragmented living tissue pieces in an emulsified state together with supplied water and remove (or collect) the fragmented living tissue pieces.

However, when the ultrasound vibration is given to the living tissue in a state in which water is supplied to near the surface, a phenomenon occurs in which the water near the surface is changed to mist containing the fragmented living tissue pieces by the ultrasound vibration and the mist scatters to surroundings.

When mist exceeding the predetermined value occurs, as shown in Fig. 7(F), the determination circuit 72 outputs a mist determination signal, for example, as shown at time t3. When the mist determination signal is outputted, the controller 73 immediately stops the water supply and stops the output of the ultrasound driving signal.

Since the distal end portion 11 of the ultrasound suction probe 8 stops ultrasonically vibrating, for example, at time t4 after a short time from time t3, the occurrence of the mist is stopped or reduced to be sufficiently small. Then, since the mist determination signal stops being outputted, the stop of the water supply is released and the stop of the output of the ultrasound driving signal is released.

As indicated by a dotted line in Fig. 7(B), the controller 73 may stop the suction in association with the stop of the water supply. In this case, the stop of the suction is released at time t4.

In a state in which water is supplied and sucked, treatment is performed by ultrasound vibration. Operation in this case is operation similar to the operation at time t2 and subsequent time points explained above. In other words, operation from time t4 to time t6 is repetition of the operation from time t2 to time t4.

When the surgeon ends the treatment by the ultrasound suction probe 8, the surgeon turns off the ultrasound switch 28a, for example, at time t7. The surgeon stops the operation of the water supply as well at time t8.

In this way, the treatment by the ultrasound suction probe 8 for the living tissue of the diseased part 5 ends.

In this embodiment in which the operation is performed as explained above, when the treatment for fragmenting and removing the fragile fat tissue in the living tissue of the diseased part 5 is performed utilizing the ultrasound vibration by the ultrasound suction probe 8, occurrence of mist exceeding the predetermined value is detected (determined) according to the image processing for an observed image. The stop or reduction of the output of the ultrasound driving signal is immediately performed according to a determination result of mist occurrence. Therefore, when mist exceeding the predetermined value occurs, it is possible to stop or reduce the mist and set the living tissue in a satisfactory observation state.

Therefore, even when the surgeon performs treatment under observation by the endoscope 14 in an environment in which mist is likely to occur, it is possible to suppress occurrence of mist exceeding the predetermined value and efficiently perform treatment by the ultrasound suction probe 8.

If the control is not performed as explained above, as explained with reference to Fig. 3B, the surgeon needs to frequently perform operation for turning off the ultrasound switch 28a in order to stop occurrence of mist that prevents observation. When the occurrence of mist is reduced or stopped, the surgeon needs to perform operation for turning on the ultrasound switch 28a again.

In contrast, according to this embodiment, occurrence of mist exceeding the predetermined value is monitored and, when occurrence of mist exceeding the predetermined value is detected, the output of the ultrasound driving signal is automatically stopped (or reduced) and the water supply operation is also stopped (or reduced) such that the occurrence of the mist is reduced or stopped. The suction operation can also be associated with the water supply operation.

In this embodiment, when a state in which an amount of occurrence of mist is equal to or smaller than the predetermined value is detected, the stop of the output of the ultrasound driving signal is automatically released and the stop of the operation of the water supply and the like is also released. Therefore, according to this embodiment, in a state with high operability in which it is unnecessary to perform troublesome operation, the surgeon can smoothly and efficiently perform the treatment by the ultrasound suction probe 8 as explained above.

In the above explanation, when the reference image and the observed image are compared to perform determination, the image signal Ira of the reference image added with the threshold Vth and the image signal Io of the observed image are compared to perform the determination. Instead of performing such determination, as shown in Fig. 8, a maximum value Vm in the image signal Ir of the reference image and the image signal Io of the observed image shown in Fig. 5C may be compared to perform the determination.

Alternatively, as shown in Fig. 8, a maximum value Vmr of the image signal Ir of the reference image added with the threshold Vth may be set and the maximum value Vmr and the image signal Io of the observed image may be compared to perform the determination. Such setting can be performed according to, for example, instruction operation for selection setting by the setting section 74.

A function in a first modification of this embodiment is explained. In the basic function included in the first embodiment explained above, in the processor 17, the mist determining and determination signal generating section 68 that determines presence or absence of occurrence of mist from an observed image is provided.

In this modification, an adhesion determining section (or an unclarity determining section) 81 is further provided that determines (detects), from an image signal of an observed image, a phenomenon in which the observed image becomes unclear because of adhesion of deposit due to mist on the outer surface of the objective lens 49.

When the adhesion determining section 81 determines that adhesion is present, the adhesion determining section 81 outputs an adhesion determination signal to the controller 73. When the adhesion determination signal is inputted, the controller 73 performs control to perform gas supply and water supply operation by the gas supply and water supply unit 55 provided in the light source unit 18.

Fig. 9A shows a schematic configuration of the adhesion determining section 81. According to a control signal from the controller 73, an image signal of the memory 65 is stored in a reference memory 81 b via a frequency/luminance analysis circuit 81a having a function of an image acquiring section.

The controller 73 outputs the control signal, for example, at time when the ultrasound switch 28a is turned on for the first time. It is likely that mist occurs after this time. However, at the starting time, the objective lens 49 of the observation window can be regarded as being in a clean state in which mist does not adhere to the objective lens 49.

At this time, the frequency/luminance analysis circuit 81a captures an image signal serving as a reference image for one frame of the memory 65, divides an image region for one frame into small regions, performs an analysis of a frequency and luminance in the respective small regions, and stores data of the analysis in the reference memory 81 b as reference analysis data.

The reference analysis data of the reference memory 81b is outputted to a comparison circuit 81 c. After the storage of the reference analysis data, the frequency/luminance analysis circuit 81a captures an image signal of an observed image for each one frame, for example, after the ultrasound switch 28a is turned off, divides an image region for each one frame into small regions Rs as shown in Fig. 9B, performs an analysis of a frequency and luminance in the respective small regions Rs, and outputs data of the analysis to the comparison circuit 81c.

The comparison circuit 81c performs comparison of frequency data and a luminance distribution between reference analysis data and analysis data in small regions corresponding thereto. When mist or the like adheres to the outer surface of the objective lens 49, an original image-forming function by the objective lens 49 is deteriorated because of deposit. Therefore, in a distribution of a spatial frequency of an acquired observed image, compared with a state without deposit, high-frequency components decrease and low-frequency components increase.

Similarly, concerning luminance components, compared with the state without deposit, unclear image components increase because of the deposit. Therefore, a difference between a maximum luminance value and a minimum luminance value decreases. Such a characteristic or tendency temporally continues.

Taking these characteristics into account, when a value on an analysis data side is equal to or smaller than a value set in advance compared with high-frequency components of the frequency data of the reference analysis data and a difference value between a maximum value and a minimum value of luminance, in other words, a contrast value, the comparison circuit 81 c outputs a comparison signal indicating that adhesion is likely to be present to the determination circuit 81d.

For example, when the number of comparison signals inputted within a predetermined time is equal to or lager than a threshold 81e, the determination circuit 81d outputs an adhesion determination signal indicating determination that adhesion is present to the controller 73. When the adhesion determination signal is inputted, the controller 73 performs control to perform gas supply and water supply operation by the gas supply and water supply unit 55. According to the control, gas and water are spouted to the outer surface of the objective lens 49 from the nozzle 50 functioning as the fluid spouting section, deposit such as scatters of mist adhering to the outer surface is removed, and the outer surface of the objective lens is set in a clean state.

When the adhesion determination signal is inputted, the controller 73 may further perform control for reducing or stopping supply of fluid by the water supply and suction unit 12 functioning as the fluid supplying section together with control for stopping or reducing output of the ultrasound driving signal by the ultrasound driving signal generating unit 6. The controller 73 may associate operation of suction with operation of water supply and perform control for reducing or stopping suction.

In the above explanation, for example, the example in which an observed image of one frame is divided into plural regions is explained. However, as a predetermined region for determining whether adhesion is present, the determination may be performed in an observed image for one frame.

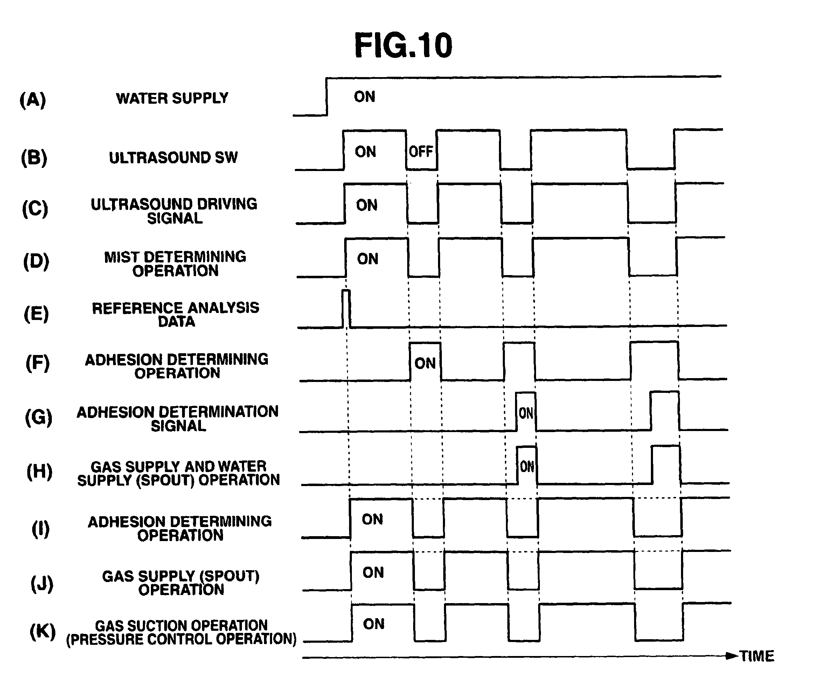

An operation example by the adhesion determining section 81 explained above is shown in Figs. 10(A) to 10(G). After water supply operation by the water supply device 41 shown in Fig. 10(A), in synchronization with an ultrasound ON signal by the ultrasound switch 28a shown in Fig. 10(B), an ultrasound driving signal shown in Fig. 10(C) is applied on the ultrasound transducer 9 of the ultrasound suction probe 8.

As shown in Fig. 10(D), during an ON period of the ultrasound switch 28a, mist determination operation by the mist determining and determination signal generating section 68 is performed.

In synchronization with the ultrasound ON signal by the ultrasound switch 28a explained above, as shown in Fig. 10(E), reference analysis data of a reference image is generated and stored in the reference memory 81b.

As shown in Fig. 10(F), in a period in which the ultrasound switch 28a is turned off, the adhesion determining section 81 performs operation for adhesion determination.

Specifically, after deciding the reference analysis data in Fig. 10(E), the adhesion determining section 81 acquires an observed image from the memory 65 and generates analysis data in a period in which the ultrasound switch 28a is turned off as shown in Fig. 10(B). Further, the adhesion determining section 81 compares the reference analysis data and the analysis data and starts the operation for adhesion determination.

For example, when the determination circuit 81d generates an adhesion determination signal as shown in Fig. 10(G), according to control by the controller 73, as shown in Fig. 10(H), the gas supply and water supply unit 55 spouts gas and water from the nozzle 50 and sets the outer surface of the objective lens 49 in a clean state.

According to this embodiment, in a state in which, because of occurrence of mist, the mist scatters and adheres to the outer surface of the objective lens 49 and a clear observed image cannot be obtained, it is possible to automatically spout gas and water from the nozzle 50 and set the outer surface of the objective lens 49 in a clean state.

In the above explanation, the example in which the operation for adhesion determination is performed in the period in which the ultrasound switch 28a is turned off as shown in Fig. 10(F) is explained.

However, the operation for adhesion determination is not limited to such a case. For example, as shown in Fig. 10(I), the operation for adhesion determination may be performed in a period in which the ultrasound switch 28a is turned ON. In this case, the adhesion determination and the operation of the mist determining and determination signal generating section 68 are simultaneously performed.

As indicated by a dotted line of Fig. 10(I), the operation for adhesion determination may be performed in a period in which the ultrasound switch 28a is turned off (not only in the period in which the ultrasound switch 28a is turned on).

A function of a second modification in this embodiment is explained.

In the first modification, it is determined from an observed image whether mist adheres to the outer surface of the objective lens 49. In the case of a determination result indicating that mist adheres, gas and liquid as fluid are spouted by the gas supply and water supply unit 55 functioning as the fluid spouting section to remove deposit.

This modification further includes means for preventing deposit due to mist from adhering to the outer surface of the objective lens 49.

Specifically, since it is likely that mist occurs because of ultrasound vibration by the ultrasound suction probe 8, as shown in Fig. 10(J), the gas supply and water supply unit 55 is caused to perform operation for supplying gas in synchronization with a period in which the ultrasound switch 28a is turned on.

By supplying the gas, the gas is spouted from the nozzle 50 to the outer surface of the objective lens 49 to prevent mist from adhering to the outer surface of the objective lens.

In this case, since atmospheric pressure in the abdomen 4 increases, as shown in Fig. 10(K), the gas is sucked by the pneumoperitoneum device 25 in the pneumoperitoneum unit 24 to perform pressure control to keep the atmospheric pressure in the abdomen 4 constant.

According to this modification, it is possible to prevent an observation field of view from being deteriorated due to mist. In this case, as indicated by a dotted line in Fig. 10(J), the gas supply operation may be performed even in a period in which the ultrasound switch 28a is turned off. In this case, it is advisable to associate the gas suction operation.

A third modification of this embodiment is explained. In this modification, in the configuration shown in Figs. 1 and 2, heating means is further provided on the distal end side of the endoscope 14 and combined with the functions by the fluid spouting section and the like explained above. In a heated state, a fat tissue included in mist is liquefied or changes to an easily removable state (compared with the case of unheated temperature).

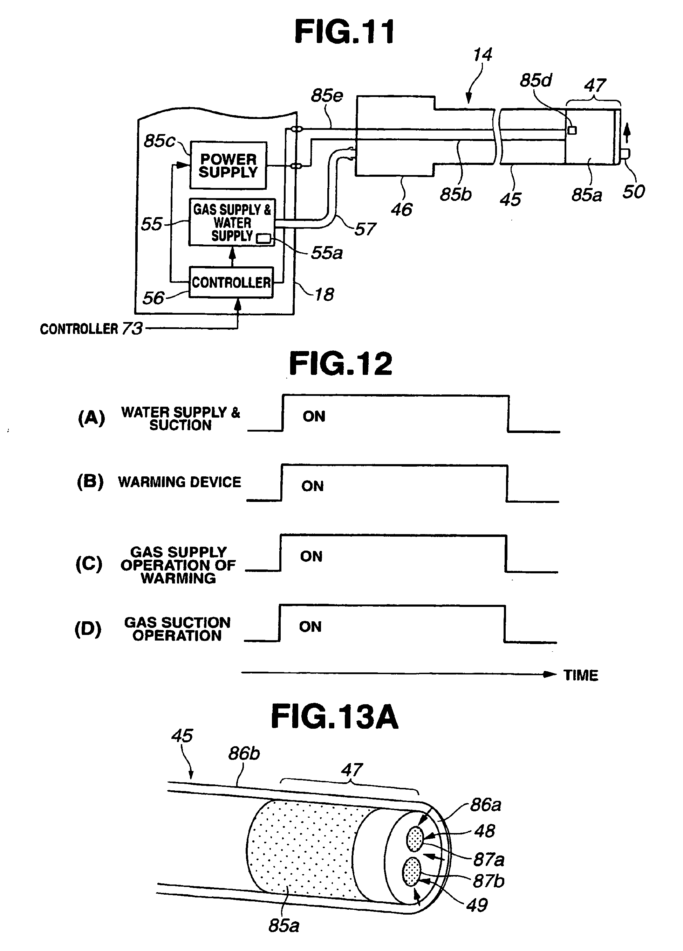

Therefore, as shown in Fig. 11, a heating device 85a that heats the vicinity of the distal end portion 47 is provided near the distal end portion 47 of the insertion portion 45 of the endoscope 14. The heating device 85a is formed in a cylindrical portion forming an outer circumferential face of the distal end portion 47 and is connected to a power supply circuit 85c for heating, which is provided in the light source unit 18, via a signal cable 85b inserted through the insertion portion 45. Operation of the power supply circuit 85c is controlled by the controller 56 or the controllers 56 and 73.

A temperature sensor 85d is provided in the heating device 85a. The temperature sensor 85d is connected to the controller 56 in the light source unit 18 via a signal cable 85e. The controller 56 performs, according to a temperature detection signal of the temperature sensor 85d, control to keep temperature for heating by the heating device 85a from the power supply circuit 85c at appropriate temperature.

For example, the controller 56 performs temperature control such that the heating device 85a has predetermined temperature T (e.g., about T=37°C to 40°C) slightly higher than body temperature. The controller 56 may directly control, according to a temperature detection signal of the temperature sensor 85d, power supply by the power supply circuit 85c to maintain the predetermined temperature.

A heater 55a that heats, for example, gas to be supplied in the gas supply and water supply unit 55 may be provided. The gas heated by the heater 55a may be supplied via the gas supply and water supply tube 57 and spouted from the nozzle 50 that projects from a distal end face of the distal end portion 47.

Figs. 12(A) to 12(D) show schematic operation explanatory diagrams in this modification.

In a period in which water supply and suction are performed to perform treatment by ultrasound vibration by the ultrasound suction probe 8 as shown in Fig. 12(A), as shown in Figs. 12(B) and 12(C), heating operation by the heating device 85a and gas supply operation for gas heated by the heater 55a are performed.

As shown in Fig. 12(D), for example, the controller 73 or 26 performs control to also perform operation of gas suction to keep the inside of the abdomen 4 at fixed pressure.

By performing such operation, it is possible to prevent mist due to ultrasound vibration from adhering to the objective lens 49. Even when mist adheres to the objective lens 49, it is possible to liquefy a fat tissue included in the mist or set the fat tissue in an easily removable state by heating of the vicinity of the distal end portion 47 and maintain an observation field of view by the objective lens 49 in a clean state.

Heated water may be supplied simultaneously with the operation for supplying the gas heated by the heater 55a. A heating device may be provided on the inside of the endoscope. When such a configuration is adopted, there is an advantage that a special additional product is unnecessary when the endoscope is used. As a place to be heated, it is advisable that, for example, at least the outer surface of the objective lens 49 can be heated.

In the above explanation, the heated gas or the like is supplied using the nozzle 50 projecting in one place from the distal end face of the distal end portion 47. However, the supply of the gas is not limited to the case of the configuration in which the nozzle 50 is used.

Fig. 13A shows an example in which a gas supply port (or a spout port) 86a different from the nozzle 50 is provided. For example, a semicylindrical gas supply pipe 86b is provided along an outer circumferential face of the insertion portion 45. The gas supply pipe 86b functions as the gas supply port 86a opened in a semicylindrical shape on the distal end face of the distal end portion 47.

The gas supply port 86a is opened to be opposed to the distal end face. Supplied gas is spouted along the distal end face as indicated by arrows. The objective lens 49 and the illumination lens 48 are exposed on the distal end face.

Therefore, heated gas is spouted to make it easy to remove, to a circumferential edge side on an opposite side of the gas supply port 86a, deposit due to mist containing a fat tissue adhering to, for example, outer surfaces of the objective lens 49 and the illumination lens 48.

In Fig. 2 and the like, the nozzle 50 is provided at the distal end portion of the gas supply and water supply tube 58 provided on an inside of the insertion portion 45 of the endoscope 14. However, as shown in Fig. 13B, a gas supply port (or nozzle) 86d may be provided at a distal end portion of the gas supply pipe 86c provided along the outer circumferential face of the insertion portion 45.

Heated gas may be spouted or delivered from the gas supply port 86d.

As shown in Figs. 13A and 13B, water repellent films 87a and 87b having a water repelling function may be provided on the outer surfaces of the objective lens 49 and the illumination lens 48. By providing the water repellent films 87a and 87b, it is possible to prevent mist from easily adhering to the outer surfaces and, even when mist adheres to the outer surfaces, it is possible to easily remove the mist.

### (Second Embodiment)

Fig. 14A shows a configuration of an ultrasound suction probe 8B according to a second embodiment of the present invention. This embodiment includes means for preventing, even when mist scatters, the scatter from affecting a satisfactory observation field of view of the endoscope 14. Other components are the same as, for example, those in the first embodiment.

In the ultrasound suction probe 8B, the outer pipe 31 of the ultrasound suction probe 8B shown in Fig. 2 is replaced with an inner sheath 91 a and an outer sheath 91 b is provided on an outer side of the inner sheath 91 a.

A distal end of the outer sheath 91b is located further on a rear side than a distal end of the inner sheath 91a. The distal end portion 11 of the ultrasound suction probe 8B arranged on an inner side of the inner sheath 91a is arranged to slightly project from a distal end portion of the inner sheath 91 a.

In a distal end side portion between the inner sheath 91a and the outer sheath 91b, an openable and closable umbrella 92a is arranged to be movable in an axis direction.

An operation lever 92b movable to the front is provided in the gripping portion 46 to which a rear end of the outer sheath 91b is fixed. A surgeon can project the umbrella 92a retracted in a distal end portion of the outer sheath 91b to project as shown in Fig. 14B by performing operation for moving (pressing) the operation lever 92b to a front side.

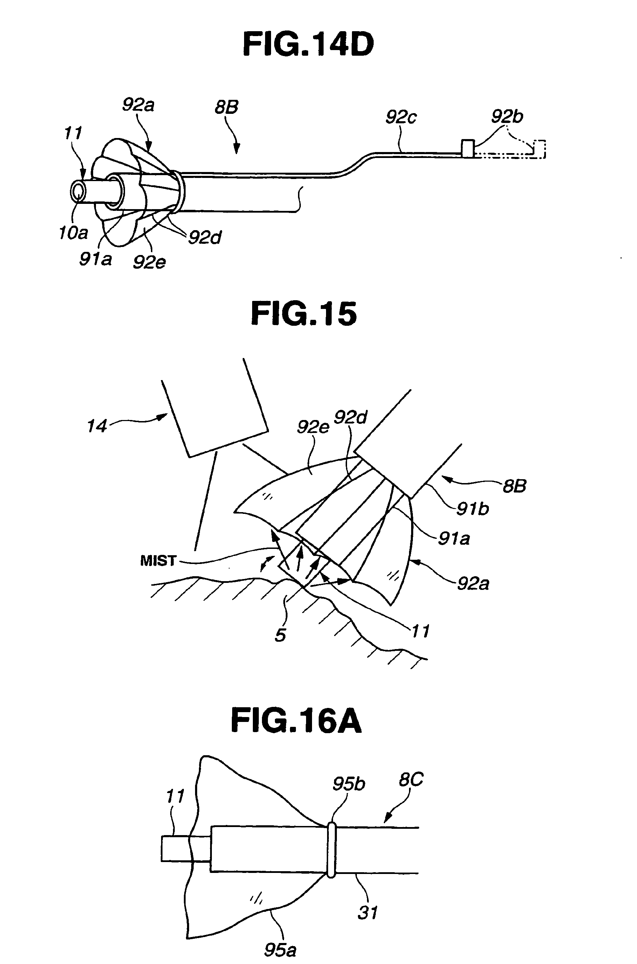

As shown in Fig. 14C, the umbrella 92a is coupled to the operation lever 92b via a slide bar 92c as it is seen from a structure in which the outer sheath 91b is detached. Therefore, the surgeon can project the umbrella 92a as shown in Figs. 14B and 14D by performing operation for moving the operation lever 92b to the front side as explained above.

The umbrella 92a includes a transparent sheet 92e formed in a substantially conical shape such that a distal end side expands compared with a proximal end side and plural wire-like framework sections 92d provided along an axis direction of the sheaths 91a and 91b to reinforce the transparent sheet 92e. For example, a proximal end of the framework sections 92d is a fixed ring section. The framework sections 92d are formed of shape memory metal or the like to have a characteristic that a distal end side of the framework sections 92d expands. Soil such as mist adhering to the umbrella 92a is periodically cleaned by perfusing water to a gap between the inner sheath 91a and the outer sheath 91 b.

Fig. 15 shows a state in which treatment by ultrasound vibration is applied to a living tissue of the diseased part 5 using the ultrasound suction probe 8B having such a configuration. The surgeon operates the operation lever 92b and sets the umbrella 92a in an open state. Since the umbrella 92a is formed by the transparent sheet 92e, the surgeon can observe a peripheral portion of the diseased part 5 seeing through the transparent sheet 92e.

When ultrasound vibration is given to the living tissue by the distal end portion 11 of the ultrasound suction probe 8B, since water is supplied to a surface of the living tissue, by giving the ultrasound vibration, fragmented living tissue pieces scatter to surroundings as mist while being mixed in the water. In Fig. 15, the scattering mist is indicated by arrows.

Even if the mist scatters, the mist can be prevented from scattering to an observation field of view side of the endoscope 14 using the umbrella 92a. Therefore, the surgeon can smoothly perform treatment by the ultrasound suction probe 8B under observation by the endoscope 14.

By periodically perfusing water to the gap between the inner sheath 91a and the outer sheath 91 b as explained above, even if an inner side of the umbrella 92a is soiled by mist, it is possible to remove the soil and maintain a state in which the living tissue can be easily observed by the endoscope 14 through the transparent sheet 92e of the umbrella 92a.

### (Third Embodiment)

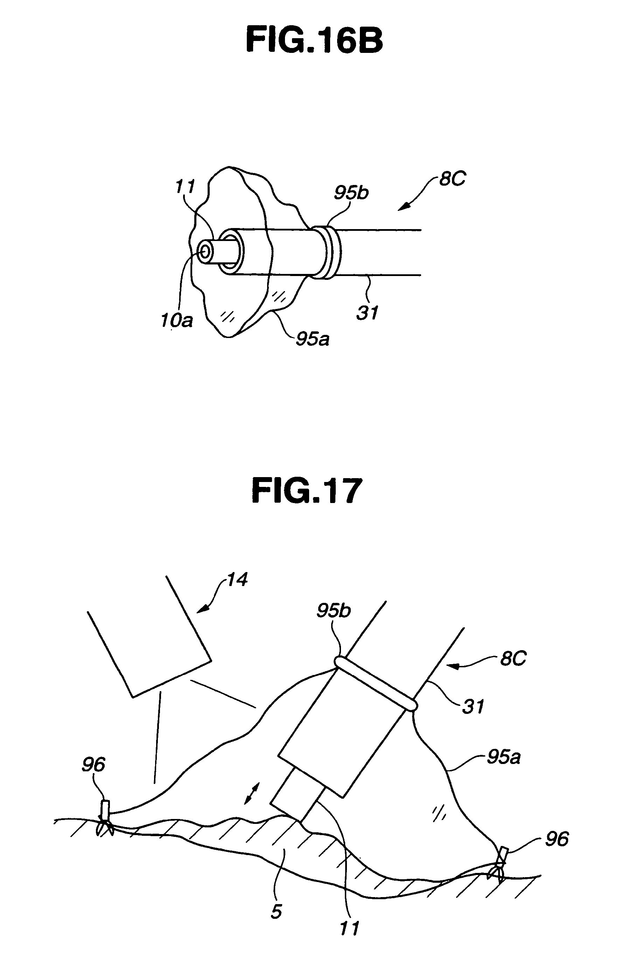

Figs. 16A and 16B show a configuration on a distal end side of an ultrasound suction probe 8C according to a third embodiment of the present invention.

In this embodiment, for example, in the ultrasound suction probe 8 shown in Figs. 1 and 2 in the first embodiment, the ultrasound suction probe 8C is formed by detachably attaching a bag 95a, which is formed of, for example, a transparent member, on the distal end side of the ultrasound suction probe 8.

The bag 95a is formed in a substantially semispherical shape or a conical shape. A proximal end of the bag 95a is detachably attached to the outer pipe 31 of the ultrasound suction probe 8 by a ring 95b having elasticity such as rubber. A distal end side of the bag 95a is opened in a substantially circular shape.

Fig. 17 shows a state in which processing utilizing ultrasound vibration is performed using the ultrasound suction probe 8C in this embodiment.

In this embodiment, a peripheral portion of the treatment target diseased part 5 is set to be on an inner side of the bag 95a under observation by the endoscope 14. Under observation by the endoscope 14, the surgeon fixes, via a not-shown treatment instrument, plural places at an opened circumferential edge of the bag 95a to the surface of the living tissue opposed to the places with clips 96.

Thereafter, as in the case of the embodiments explained above, the surgeon performs treatment with the ultrasound suction probe 8C. In the case of this embodiment, even if mist scatters, it is possible to effectively prevent the mist from scattering from an inside of the bag 95a to an outside. It is possible to observe the peripheral portion of the diseased part 5 seeing through the transparent bag 95a using the endoscope 14.

Therefore, it is possible to smoothly perform the treatment by the ultrasound suction probe 8C under observation by the endoscope 14.

A configuration or a method obtained by modifying the embodiments or the modifications explained above may be adopted.

For example, in the explanation of the first modification of the first embodiment, as shown in Figs. 10(A) to 10(C), when the ultrasound switch 28a is turned on, an ultrasound driving signal is continuously outputted.

On the other hand, an output mode setting section 74b may be provided in the setting section 74 and an ultrasound driving signal may be intermittently outputted according to selection setting by the output mode setting section 74b.

### (Fourth Embodiment)

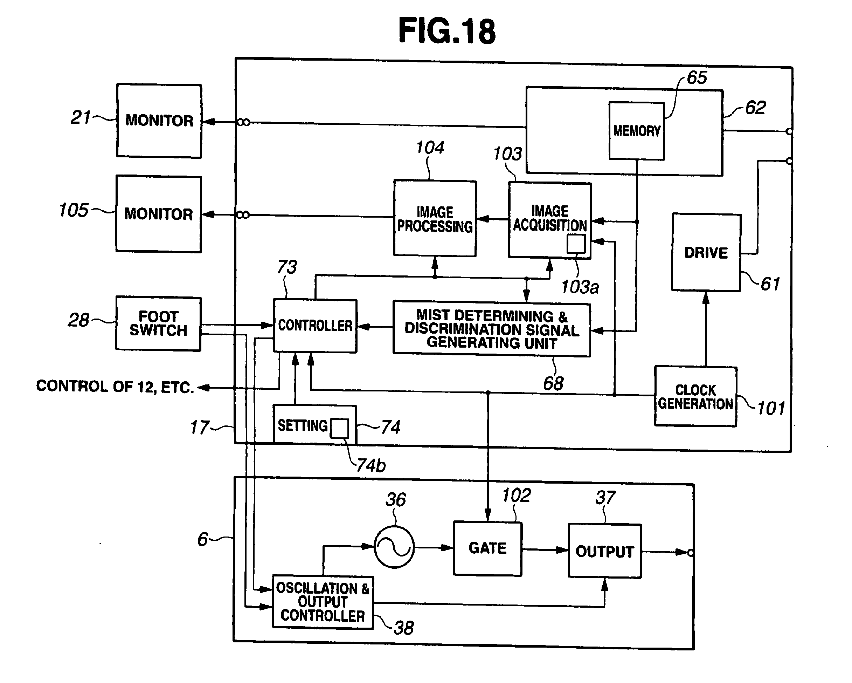

Fig. 18 shows a configuration of the processor 17 and the ultrasound driving signal generating unit 6 in an ultrasound suction system according to a fourth embodiment of the present invention. In this embodiment, as shown in Fig. 18, a clock generation circuit 101, a gate circuit 102, an image acquisition circuit 103, an image processing circuit 104, and a monitor 105 are further provided in the first embodiment.

In this embodiment, when the ultrasound switch 28a is turned on, opening and closing of the gate circuit 102 in the ultrasound driving signal generating unit 6 is controlled according to a clock of the clock generation circuit 101 provided in the processor 17. An output signal of the oscillator 36 is intermittently outputted to the output circuit 37 according to the opening and closing of the gate circuit 102. As the clock generation circuit 101, the clock circuit 69a shown in Fig. 4 may be used.

An image signal of the memory 65 is outputted to the second monitor 105 via the second image acquisition circuit 103 that acquires an image in synchronization with a clock and the image processing circuit 104 that performs image processing.

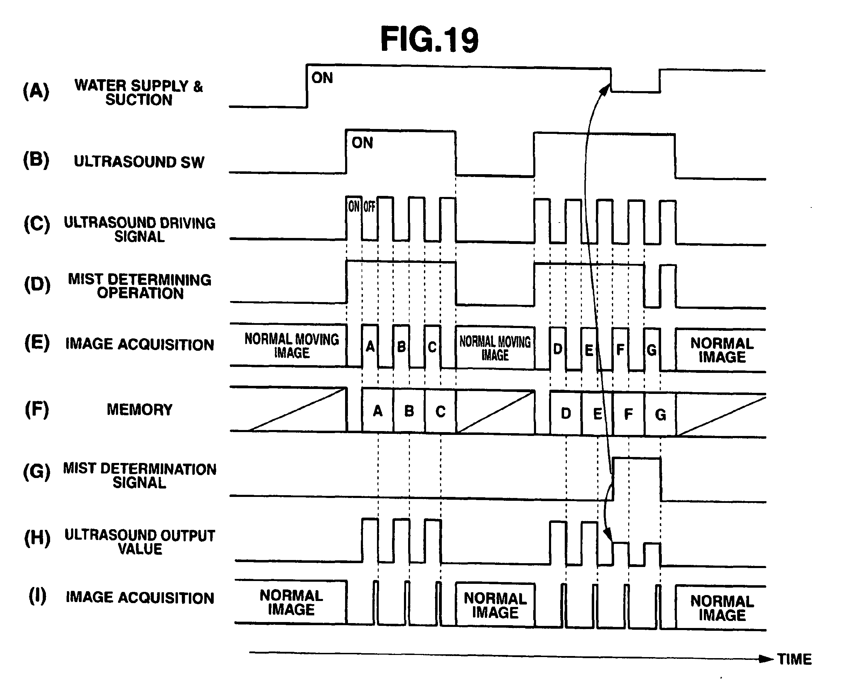

The second image acquisition circuit 103 acquires the image signal from the memory 65 in a period in which an ultrasound driving signal is turned off (see Fig. 19(E)) and freezes and stores the image signal in a memory 103a in a period in which the ultrasound driving signal is turned on (see Fig. 19(F)).

When an image signal acquired in a period in which a next ultrasound driving signal is turned off is inputted, the image signal stored in the memory 103a is updated. An output signal of the second image acquisition circuit 103 is converted into a standard image signal by the image processing circuit 104 and outputted to the monitor 105.

An observed image acquired by the second image acquisition circuit 103 is displayed on the monitor 105. When the ultrasound switch 28a is not turned on, the image acquisition circuit 103 acquires a moving image from the memory 65 at a normal frame rate. The moving image is displayed on the monitor 105.

The second image acquisition circuit 103 and the image processing circuit 104 are controlled by the controller 73. The output mode setting section 74b is provided in the setting section 74. It is possible to select, according to selection setting by the output mode setting section 74b, one output mode from the continuous output mode and an intermittent output mode explained below as in the first embodiment.

When the continuous output mode is selected, the gate circuit 102 is usually open. Operation same as that in the first embodiment is performed.

Other components are the same as those in the first embodiment shown in Fig. 2. Figs. 19(A) to 19(F) show timing charts for operation explanation in the case in which the intermittent output mode in this embodiment is selected.

After water supply and suction operation is started as shown in Fig. 19(A), as shown in Fig. 19(B), the ultrasound switch 28a is turned on and off to perform treatment utilizing ultrasound vibration by the ultrasound suction probe 8.

In this embodiment, when the ultrasound switch 28a is turned on, an ultrasound driving signal is intermittently outputted as shown in Fig. 19(C) in synchronization with a clock. As shown in Fig. 19(D), the mist determining and determination signal generating section 68 starts mist determination operation in a period in which the ultrasound switch 28a is turned on as explained in the first embodiment.

As shown in Fig. 19(E), the second image acquisition circuit 103 acquires, in synchronization with the ultrasound driving signal outputted intermittently, an observed image in a period in which the ultrasound driving signal is off. A standard image signal generated according to the observed image is displayed on the monitor 105. In a period in which the ultrasound switch 28a is off, as shown in Fig. 19(E), an observed image of a moving image, i.e., a normal moving image is acquired at a predetermined frame rate (e.g., 20 frames/sec or 30 frames/sec).

In Fig. 19(E), when intermittently-acquired observed images are represented as A, B, C, ..., F, and G, as shown in Fig. 19(F), for example, the acquired observed images are stored in the memory 103a at a double period. The observed images are displayed as a moving image at a double period (in this example, a period of ON/OFF of the ultrasound driving signal) on the monitor 105.

The period of ON/OFF of the ultrasound driving signal may be able to be variably set by the setting section 74. Consequently, the surgeon may be able to select, according to treatment, a period in which the ultrasound driving signal is turned on and a period in which the ultrasound driving signal is turned off in the intermittent output mode. Duties in the period in which the ultrasound driving signal is turned on and the period in which the ultrasound driving signal is turned off may be able to be variably set by the setting section 74.

When each of the intermittently-acquired observed images A and the like in Fig. 19(E) is plural frames, an average of the frames is set in an observed image of one frame. As shown in Fig. 19(I) explained later, only an observed image of one frame or one field may be acquired. In the case of a normal image in Fig. 19(F), observed images are recorded in the memory 103a at the predetermined frame rate. In Fig. 19(F), operation of the recording is indicated by diagonal lines.

In this embodiment, when the ultrasound switch 28a is turned on, the ultrasound driving signal is intermittently outputted and observed images acquired in periods in which the ultrasound driving signal is not outputted are displayed, on the monitor 105 functioning as display means, as a moving image at a low frame rate equal to or lower than 1/2 of the normal frame rate for a moving image.

Therefore, even under a condition in which mist is likely to occur because of ultrasound vibration, since observed images are acquired and displayed in periods in which the ultrasound vibration stops, it is possible to display, on the monitor 105, observed images in a state in which likelihood of observed images becoming images less easily observed due to occurrence of mist exceeding the predetermined value is at least reduced.

For example, as shown in Fig. 19(G), when a mist determination signal indicating determination of occurrence of mist exceeding the predetermined value is outputted by the mist determining and determination signal generating section 68, as shown in Fig. 19(H), the controller 73 reduces an ultrasound output value outputted from the output circuit 37. The controller 73 reduces the operation of water supply and suction of the water supply and suction unit 12 shown in Fig. 19(A).

Quality of an observed image displayed on the monitor 21 side as in the first embodiment is maintained. In Fig. 19(H), an example in which an ultrasound output value is reduced when a mist determination signal is outputted is explained. However, as shown in Fig. 7(D), the ultrasound output value may be set to 0, i.e., the output of the ultrasound driving signal may be stopped.

In the period in which the ultrasound driving signal is off as in Fig. 19(E), instead of acquiring images of plural frames or plural fields, as shown in Fig. 19(I), an image of one frame or one field immediately before the ultrasound driving signal in the period in which the ultrasound driving signal is off is turned on may be acquired.

Consequently, even under a condition in which mist occurs because of ultrasound vibration, it is possible to acquire observed images in which likelihood of observed images becoming less easily observed due to occurrence of mist exceeding the predetermined value is reduced and display the observed images on the monitor 105.

This embodiment has effects same as those of the first embodiment. Besides, it is possible to display, on the second monitor 105, observed images with little adverse effect due to occurrence of mist exceeding the predetermined value. Therefore, the surgeon can smoothly perform treatment by ultrasound vibration.

As a modification of this embodiment, operation for, until a mist determination signal is generated, outputting the ultrasound driving signal in the continuous output mode as explained in the first embodiment and, after the mist determination signal is generated, changing the continuous output mode to the intermittent output mode may be selected or controlled.

In the embodiments explained above, the configuration including, in the ultrasound suction probe 8, the ultrasound transducer 9 functioning as the ultrasound generating section that generates ultrasound vibration is explained. However, a configuration in which the ultrasound generating section is provided on the outside of the ultrasound suction probe 8 may be adopted. In Figs. 2 and the like, the controller 73 is shown as, for example, a component separate from the mist determining and determination signal generating section 68. However, a configuration in which the controller 73 includes the mist determining and determination signal generating section 68 may be adopted. A configuration in which the controller 73 includes the adhesion determining section 81 may be adopted.

The embodiments and the like explained above may be partially combined to form a different embodiment or form a modification.

## Claims

1. An ultrasound suction system (1) comprising:
an ultrasound driving signal generating section (6) adapted to generate an ultrasound driving signal;
an ultrasound suction section (8) including an ultrasound generating section adapted to (9) generate ultrasound vibration energy according to application of the ultrasound driving signal and a suction section (10a) adapted to give the ultrasound vibration energy generated by the ultrasound generating section (9) to a treatment target living tissue from a distal end portion (11) of a vibration transmitting section (10), adapted to transmit the ultrasound vibration energy to the treatment target living tissue, to thereby fragment the living tissue, and sucking fragmented living tissue pieces; and
an observing section (15) that has an observation function for observing the living tissue opposed to the distal end portion (11) side of the vibration transmitting section (10),
a fluid supplying section (41) that supplies fluid to a surface of the living tissue; **characterized in that** the ultrasound suction system (1) further comprises
an image acquiring section (69) adapted to acquire, via the observing section (15), an observed image in which mist is generated from the surface of the living tissue in a state in which the ultrasound vibration energy is given; and
a control section (73) adapted to control at least one of output of the ultrasound driving signal by the ultrasound driving signal generating section (6) and supply of the fluid on the basis of a comparison result obtained by comparing: a reference image corresponding to a state in which the mist is not substantially generated by the ultrasound driving signal from the time of giving the ultrasound vibration energy to the distal end portion (11) of the vibration transmitting section (10) to the time immediately after the giving of the ultrasound vibration energy; and the observed image acquired by the image acquiring section (69).

2. The ultrasound suction system (1) according to claim 1, further comprising a determining section (72) that determines, on the basis of the comparison result, whether the observed image is an observed image in a state in which mist that has changed by a predetermined value or more from the reference image is generated, wherein, in a case of a result of determination that the mist has changed by the predetermined value or more, the control section (73) performs control for reducing or stopping the supply of the fluid by the fluid supplying section (41) together with control for stopping or reducing output of the ultrasound driving signal by the ultrasound driving signal generating section (6).

3. The ultrasound suction system (1) according to claim 2, further comprising a suction device (42) for sucking the fragmented living tissue pieces together with the fluid supplied to the surface of the living tissue via a suction tube (13b) that communicates with the suction section (10a), wherein
when a determination result is obtained that the observed image has changed by a predetermined value or more from the reference image on the basis of a result of comparison between the reference image and the observed image, the control section (73) performs control for reducing or stopping the sucking operation by the suction device (42).

4. The ultrasound suction system (1) according to claim 1, wherein the observing section (15) includes an endoscope (14) provided at a distal end portion of an insertion portion (45).

5. The ultrasound suction system (1) according to claim 2, wherein the image acquiring section (69) sets, as the reference image, an observed image acquired from the observing section (15) in a state in which the ultrasound driving signal is not substantially generated, and the determining section (72) determines presence or absence of generation of the mist by performing, with respect to the reference image, determination whether the observed image acquired from the observing section (15) in a state in which the ultrasound driving signal is generated includes a pulse-like image signal as an image signal corresponding to the generation of the mist.

6. The ultrasound suction system (1) according to claim 5, wherein the determining section (72) determines presence or absence of generation of the mist by determining whether the observed image is an image including a luminance value equal to or larger than a threshold set in advance compared with the reference image.

7. The ultrasound suction system (1) according to claim 6, wherein the determining section (72) determines whether the observed image discretely includes a predetermined number or more of luminance values equal to or larger than the threshold in a predetermined region compared with the reference image and, when the observed image includes the predetermined number or more of luminance values, generates a mist determination signal.

8. The ultrasound suction system (1) according to claim 5, further comprising an adhesion determining section (81) that determines from the observed image whether a deposit involved in scattering of the mist adheres on an outer surface of a distal end portion of the observing section (15), wherein the adhesion determining section (81) generates a determination signal when the adhesion determining section determines that the deposit has adhered on the outer surface.

9. The ultrasound suction system (1) according to claim 8, wherein the control section (73) performs, on the basis of the generation of the determination signal, control for spouting the fluid from a fluid spouting section (50) provided at the distal end portion (47) of an endoscope (14) to which the observing section (15) is provided to an outer surface of the distal end portion of the observing section (15).

10. The ultrasound suction system (1) according to claim 9, wherein the adhesion determining section (81) generates the determination signal in a case of a result of determination that a difference between a maximum and a minimum of luminance values in the observed image is equal to or smaller than a predetermined value.

11. The ultrasound suction system (1) according to claim 9, wherein the adhesion determining section (81) includes an analyzing section (81a) that analyzes a spatial frequency distribution in the observed image, and the adhesion determining section generates the determination signal in a case of a result of determination that a high-frequency component of the spatial frequency is equal to or smaller than a predetermined value.

12. The ultrasound suction system (1) according to claim 5, wherein the control section (73) further performs control for spouting gas from a gas spouting section (50) provided at the distal end portion (47) of an endoscope (14) to which the observing section (15) is provided to an outer surface of a distal end portion of the observing section (15) at least in a period in which the ultrasound driving signal is generated.

13. The ultrasound suction system (1) according to claim 12, further comprising
a pneumoperitoneum unit (24) that supplies and sucks gas to and from a body cavity, into which at least a distal end side of the endoscope (14) is inserted, wherein
the control section (73) controls operation of at least the gas suction in the pneumoperitoneum unit (24) on the basis of pressure information of the gas in the body cavity.

14. The ultrasound suction system (1) according to claim 2, further comprising:
a heating device (85a) that is provided at a distal end portion (47) of an endoscope (14) to which the observing section (15) is provided and heats a vicinity of the distal end portion (47); and
a power supply section (85c) that supplies electric power for heating to the heating device (85a).

15. The ultrasound suction system (1) according to claim 10, wherein, in a case where the determination signal is generated, the control section (73) performs control for reducing or stopping the supply of the fluid by the fluid supplying section (41) together with control for stopping or reducing output of the ultrasound driving signal by the ultrasound driving signal generating section (6).

16. The ultrasound suction system (1) according to claim 2, further comprising
a transparent umbrella (92a) provided so as to be capable of projecting and retracting on an inner side of an overcoat sheath on a distal end side of the ultrasound suction section (8), wherein, when projected, the umbrella (92a) covers a periphery of a distal end portion of an ultrasound suction section (8) with a transparent sheet (92e) having a substantially conical shape.

17. The ultrasound suction system (1) according to claim 2, further comprising
a transparent bag (95a), a proximal end of which is detachably provided and a distal end side of which is opened, on a distal end side of the ultrasound suction section (8), wherein an opened circumferential edge of the bag (95a) can be fixed by a clip (96) to a living tissue around a distal end portion of the ultrasound suction section (8).

18. The ultrasound suction system (1) according to claim 2, wherein the ultrasound driving signal generating section (6) has a continuous output mode for continuously outputting the generated ultrasound driving signal and an intermittent output mode for intermittently outputting the ultrasound driving signal at a predetermined period including an ON period and an OFF period.

19. The ultrasound suction system (1) according to claim 2, the control section (73) performs control including reduction of the output of the ultrasound driving signal by controlling a current value of the ultrasound driving signal outputted by the ultrasound driving signal generating section.

## Patentansprüche

1. Ultraschall-Saugsystem (1) mit:
einer Ultraschall-Antriebssignal-Erzeugungseinheit (6), die ein Ultraschall-Antriebssignal zu erzeugen vermag;
einer Ultraschall-Saugeinheit (8) mit einer Ultraschall-Erzeugungseinheit (9), die dazu eingerichtet ist, Ultraschall-Schwingungsenergie entsprechend dem Anlegen des Ultraschall-Antriebssignals zu erzeugen, und einer Saugeinheit (10a), die dazu eingerichtet ist, die durch die Ultraschall-Erzeugungseinheit (9) erzeugte Ultraschall-Schwingungsenergie von einem distalen Endbereich (11) einer Schwingungsübertragungseinheit (10), die dazu eingerichtet ist, die Ultraschall-Schwingungsenergie zu einem lebenden Gewebe des Behandlungsziels zu übertragen, an das lebende Gewebe des Behandlungsziels zu übergeben, um dadurch das lebende Gewebe zu zerteilen und abgetrennte Stücke des lebenden Gewebes abzusaugen;
einer Beobachtungseinheit (15), die eine Beobachtungsfunktion zum Beobachten des lebenden Gewebes aufweist, welches der Seite des distalen Endbereichs (11) der Schwingungsübertragungseinheit (10) gegenüberliegt; und
einer Fluidzuführeinheit (41), die einer Oberfläche des lebenden Gewebes Fluid zuführt;
**dadurch gekennzeichnet, dass** das Ultraschall-Saugsystem (1) des Weiteren aufweist:
eine Bilderfassungseinheit (69), die über die Beobachtungseinheit (15) ein beobachtetes Bild zu erfassen vermag, in dem Dunst von der Oberfläche des lebenden Gewebes in einem Zustand erzeugt wird, in welchem die Ultraschall-Schwingungsenergie abgegeben wird; und
einen Steuerbereich (73), der dazu eingerichtet ist, die Ausgabe des Ultraschall-Antriebssignals durch die Ultraschall-Antriebssignal-Erzeugungseinheit (6) und/oder die Zufuhr des Fluids auf der Grundlage eines Vergleichsergebnisses zu steuern, das erzielt wird durch Vergleichen von: einem Referenzbild, das einem Zustand entspricht, in dem der Dunst von der Zeit des Abgebens der Ultraschall-Schwingungsenergie an den distalen Endbereich (11) der Schwingungsübertragungseinheit (10) bis zur Zeit unmittelbar nach dem Abgeben der Ultraschall-Schwingungsenergie im Wesentlichen nicht durch das Ultraschall-Antriebssignal erzeugt wird, und dem durch die Bilderfassungseinheit (69) erfassten beobachteten Bild.

2. Ultraschall-Saugsystem (1) nach Anspruch 1, das des Weiteren eine Bestimmungseinheit (72) aufweist, die auf der Grundlage des Vergleichsergebnisses bestimmt, ob das beobachtete Bild ein beobachtetes Bild in einem Zustand ist, in welchem Dunst erzeugt wird, der sich zum Referenzbild um einen vorgegebenen oder höheren Wert geändert hat, wobei, in einem Fall eines Bestimmungsergebnisses, bei dem der Dunst sich um den vorgegebenen oder höheren Wert geändert hat, der Steuerbereich (73) die Steuerung zum Reduzieren oder Anhalten der Zufuhr des Fluids durch die Fluidzuführeinheit (41) zusammen mit der Steuerung zum Anhalten oder Reduzieren der Ausgabe des Ultraschall-Antriebssignals durch die Ultraschall-Antriebssignal-Erzeugungseinheit (6) durchführt.

3. Ultraschall-Saugsystem (1) nach Anspruch 2, das des Weiteren eine Saugeinrichtung (42) aufweist, zum Absaugen der abgetrennten lebenden Gewebestücke zusammen mit dem Fluid, das der Oberfläche des lebenden Gewebes zugeführt wird, über einen Saugschlauch (13b), der mit der Saugeinheit (10a) in Verbindung steht, wobei
wenn ein Bestimmungsergebnis erzielt wird, bei dem beobachtete Bild sich zum Referenzbild auf der Grundlage des Ergebnisses eines Vergleichs zwischen dem Referenzbild und dem beobachteten Bild um einen vorgegebenen oder höheren Wert geändert hat, der Steuerbereich (73) die Steuerung zum Reduzieren oder Anhalten des Saugprozesses durch die Saugeinrichtung (42) durchführt.

4. Ultraschall-Saugsystem (1) nach Anspruch 1, wobei die Beobachtungseinheit (15) ein Endoskop (14) aufweist, das an einem distalen Endbereich eines Einführbereichs (45) vorgesehen ist.

5. Ultraschall-Saugsystem (1) nach Anspruch 2, wobei die Bilderfassungseinheit (69) als Referenzbild ein beobachtetes Bild festlegt, das von der Beobachtungseinheit (15) in einem Zustand erfasst wird, in welchem das Ultraschall-Antriebssignal im Wesentlichen nicht erzeugt wird, und die Bestimmungseinheit (72) feststellt, ob Dunst erzeugt wird oder nicht, indem im Hinblick auf das Referenzbild die Bestimmung durchgeführt wird, ob das beobachtete Bild, das von der Beobachtungseinheit (15) in einem Zustand erfasst wird, in dem das Ultraschall-Antriebssignal erzeugt wird, ein pulsartiges Bildsignal als Bildsignal entsprechend der Erzeugung von Dunst aufweist.

6. Ultraschall-Saugsystem (1) nach Anspruch 5, wobei die Bestimmungseinheit (72) feststellt, ob Dunst erzeugt wird oder nicht, indem sie bestimmt, ob das beobachtete Bild ein Bild mit einem Luminanzwert gleich oder größer als ein Schwellenwert ist, der im Vergleich zum Referenzbild vorab eingestellt wird.

7. Ultraschall-Saugsystem (1) nach Anspruch 6, wobei die Bestimmungseinheit (72) feststellt, ob das beobachtete Bild eine vorgegebene oder höhere Anzahl von Luminanzwerten einzeln aufweist, die gleich oder größer als der Schwellenwert in einer vorgegebenen Region im Vergleich zum Referenzbild sind, und ein Dunstbestimmungssignal erzeugt, wenn das beobachtete Bild die vorgegebene oder höhere Anzahl von Luminanzwerten aufweist.

8. Ultraschall-Saugsystem (1) nach Anspruch 5, das des Weiteren eine Anhaftung-Bestimmungseinheit (81) aufweist, die aus dem beobachteten Bild feststellt, ob eine Ablagerung, die mit dem Zerstäuben des Dunstes zu tun hat, an einer Außenfläche eines distalen Endbereichs der Beobachtungseinheit (15) anhaftet, wobei die Anhaftung-Bestimmungseinheit (81) ein Bestimmungssignal erzeugt, wenn die Anhaftung-Bestimmungseinheit feststellt, dass die Ablagerung an der Außenfläche anhaftet.

9. Ultraschall-Saugsystem (1) nach Anspruch 8, wobei der Steuerbereich (73) auf der Grundlage der Erzeugung des Bestimmungssignals eine Steuerung durchführt, zum Herausspritzen des Fluids aus einer Fluidspritzeinheit (50), die am distalen Endbereich (47) eines Endoskops (14), an dem sich die Beobachtungseinheit (15) befindet, vorgesehen ist, auf eine Außenfläche des distalen Endbereichs der Beobachtungseinheit (15).

10. Ultraschall-Saugsystem (1) nach Anspruch 9, wobei die Anhaftung-Bestimmungseinheit (81) das Bestimmungssignal im Falle eines Bestimmungsergebnisses erzeugt, bei dem eine Differenz zwischen einem Maximum und einem Minimum von Luminanzwerten in dem beobachteten Bild gleich oder kleiner als ein vorgegebener Wert ist.

11. Ultraschall-Saugsystem (1) nach Anspruch 9, wobei die Anhaftung-Bestimmungseinheit (81) eine Analyseeinheit (81a) aufweist, die eine Raumfrequenzverteilung im beobachteten Bild analysiert, und die Anhaftung-Bestimmungseinheit das Bestimmungssignal im Falle eines Bestimmungsergebnisses erzeugt, bei dem eine Hochfrequenzkomponente der Raumfrequenz gleich oder kleiner als ein vorgegebener Wert ist.

12. Ultraschall-Saugsystem (1) nach Anspruch 5, wobei der Steuerbereich (73) des Weiteren eine Steuerung durchführt, zum Heraussprühen von Gas aus einer Gassprüheinheit (50), die am distalen Endbereich (47) eines Endoskops (14), an dem sich die Beobachtungseinheit (15) befindet, vorgesehen ist, auf eine Außenfläche eines distalen Endbereichs der Beobachtungseinheit (15) zumindest in einer Zeitspanne, in der das Ultraschall-Antriebssignal erzeugt wird.

13. Ultraschall-Saugsystem (1) nach Anspruch 12, das des Weiteren aufweist:
eine Pneumoperitoneum-Einheit (24), die Gas zu einem Körperhohlraum zuführt und aus ihm absaugt, in den mindestens eine distale Endseite des Endoskops (14) eingeführt ist, wobei
der Steuerbereich (73) die Funktion zumindest der Gasabsaugung in der Pneumoperitoneum-Einheit (24) auf der Grundlage von Druckinformation über das Gas im Körperhohlraum steuert.

14. Ultraschall-Saugsystem (1) nach Anspruch 2, das des Weiteren aufweist:
eine Heizeinrichtung (85a), die an einem distalen Endbereich (47) eines Endoskops (14), an dem sich die Beobachtungseinheit (15) befindet, vorgesehen ist und eine Umgebung des distalen Endbereichs (47) heizt; und
eine Stromversorgungseinheit (85c), welche die Heizeinrichtung (85a) mit elektrischem Strom zum Heizen versorgt.

15. Ultraschall-Saugsystem (1) nach Anspruch 10, wobei in einem Fall, in dem das Bestimmungssignal erzeugt wird, der Steuerbereich (73) die Steuerung zum Reduzieren oder Anhalten der Zufuhr des Fluids durch die Fluidzuführeinheit (41) zusammen mit der Steuerung zum Anhalten oder Reduzieren der Ausgabe des Ultraschall-Antriebssignals durch die Ultraschall-Antriebssignal-Erzeugungseinheit (6) durchführt.

16. Ultraschall-Saugsystem (1) nach Anspruch 2, das des Weiteren aufweist:
einen transparenten Schirm (92a), der so vorgesehen ist, dass er auf einer Innenseite einer Ummantelung an einer distalen Endseite der Ultraschall-Saugeinheit (8) vorstehen und sich zurückziehen kann, wobei der Schirm (92a), wenn er vorsteht, eine Umgebung eines distalen Endbereichs einer Ultraschall-Saugeinheit (8) mit einer transparenten Folie (92e) bedeckt, die eine im Wesentlichen konische Form aufweist.

17. Ultraschall-Saugsystem (1) nach Anspruch 2, das des Weiteren aufweist:
einen transparenten Beutel (95a), dessen proximales Ende abnehmbar vorgesehen ist und dessen distale Endseite geöffnet ist, an einer distalen Endseite der Ultraschall-Saugeinheit (8), wobei ein geöffneter Umfangsrand des Beutels (95a) durch eine Klemme (96) an einem lebenden Gewebe um einen distalen Endbereich der Ultraschall-Saugeinheit (8) herum befestigt werden kann.

18. Ultraschall-Saugsystem (1) nach Anspruch 2, wobei die Ultraschall-Antriebssignal-Erzeugungseinheit (6) einen kontinuierlichen Ausgabemodus zum kontinuierlichen Ausgeben des erzeugten Ultraschall-Antriebssignals und einen intermittierenden Ausgabemodus zum intermittierenden Ausgeben des Ultraschall-Antriebssignals zu einer vorgegebenen Zeitdauer aufweist, die eine AN-Zeit und eine AUS-Zeit enthält.

19. Ultraschall-Saugsystem (1) nach Anspruch 2, wobei der Steuerbereich (73) die Steuerung einschließlich der Reduzierung der Ausgabe des Ultraschall-Antriebssignals durch Steuern eines Stromwertes des Ultraschall-Antriebssignals durchführt, das durch die Ultraschall-Antriebssignal-Erzeugungseinheit ausgegeben wird.

## Revendications

1. Système (1) d'aspiration par ultrasons comprenant :
une section (6) de génération de signal d'attaque ultrasonore adaptée pour générer un signal d'attaque ultrasonore ;
une section (8) d'aspiration par ultrasons contenant une section (9) de génération d'ultrasons adaptée pour générer une énergie de vibration ultrasonore conformément à l'application du signal d'attaque ultrasonore et une section (10a) d'aspiration adaptée pour délivrer l'énergie de vibration ultrasonore générée par la section (9) de génération d'ultrasons vers un tissu vivant cible de traitement depuis une partie (11) d'extrémité distale d'une section (10) de transmission de vibration, adaptée pour transmettre l'énergie de vibration ultrasonore vers le tissu vivant cible de traitement, pour de ce fait fragmenter le tissu vivant, et aspirer les morceaux de tissu vivant fragmenté ;
une section (15) d'observation qui possède une fonction d'observation destinée à observer le tissu vivant opposé au côté de la partie (11) d'extrémité distale de la section (10) de transmission de vibration ; et
une section (41) d'alimentation en fluide qui délivre du fluide vers une surface du tissu vivant ;
**caractérisé en ce que** le système (1) d'aspiration par ultrasons comprend en outre
une section (69) d'acquisition d'image adaptée pour acquérir, via la section (15) d'observation, une image observée dans laquelle de la brume est générée à partir de la surface du tissu vivant dans un état dans lequel l'énergie de vibration ultrasonore est délivrée ; et
une section (73) de commande adaptée pour commander au moins l'une parmi la sortie du signal d'attaque ultrasonore par la section (6) de génération de signal d'attaque ultrasonore et l'alimentation en fluide sur la base d'un résultat de comparaison obtenu en comparant : une image de référence correspondant à un état dans lequel la brume n'est sensiblement pas générée par le signal d'attaque ultrasonore à partir du moment où l'énergie de vibration ultrasonore est délivrée vers la partie (11) d'extrémité distale de la section (10) de transmission de vibration jusqu'au moment immédiatement après que l'énergie de vibration ultrasonore a été délivrée ; et l'image observée acquise par la section (69) d'acquisition d'image.

2. Système (1) d'aspiration par ultrasons selon la revendication 1, comprenant en outre une section (72) de détermination adaptée pour déterminer, sur la base du résultat de comparaison, si l'image observée est une image observée dans un état dans lequel la brume qui a changé d'une valeur prédéterminée ou plus par rapport à l'image de référence est générée, dans lequel, dans le cas d'un résultat de détermination que la brume a changé de la valeur prédéterminée ou plus, la section (73) de commande réalise une commande pour réduire ou arrêter l'alimentation en fluide par la section (41) d'alimentation en fluide avec une commande destinée à arrêter ou à réduire la sortie du signal d'attaque ultrasonore par la section (6) de génération de signal d'attaque ultrasonore.

3. Système (1) d'aspiration par ultrasons selon la revendication 2, comprenant en outre un dispositif (42) d'aspiration destiné à aspirer les morceaux de tissu vivant fragmenté conjointement avec le fluide délivré vers la surface du tissu vivant via un tube d'aspiration (13b) qui communique avec la section (10a) d'aspiration, dans lequel
lorsque le résultat de détermination est obtenu montrant que l'image observée a changé d'une valeur prédéterminée ou plus par rapport à l'image de référence sur la base d'un résultat de comparaison entre l'image de référence et l'image observée, la section (73) de commande réalise une commande destinée à réduire ou à arrêter l'opération d'aspiration par le dispositif (42) d'aspiration.

4. Système (1) d'aspiration par ultrasons selon la revendication 1, dans lequel la section (15) d'observation comprend un endoscope (14) prévu au niveau d'une partie d'extrémité distale d'une partie (45) d'insertion.

5. Système (1) d'aspiration par ultrasons selon la revendication 2, dans lequel la section (69) d'acquisition d'image établit, en tant qu'image de référence, une image observée acquise à partir de la section (15) d'observation dans un état dans lequel le signal d'attaque ultrasonore n'est sensiblement pas généré, et la section (72) de détermination détermine la présence ou l'absence de génération de brume en réalisant, par rapport à l'image de référence, une détermination stipulant si l'image observée acquise à partir de la section (15) d'observation dans un état dans lequel le signal d'attaque ultrasonore est généré, contient un signal d'image de type impulsionnel en tant que signal d'image correspondant à la génération de la brume.

6. Système (1) d'aspiration par ultrasons selon la revendication 5, dans lequel la section (72) de détermination détermine la présence ou l'absence de génération de brume en déterminant si l'image observée est une image contenant une valeur de luminance égale ou supérieure à un seuil établi à l'avance comparée à l'image de référence.

7. Système (1) d'aspiration par ultrasons selon la revendication 6, dans lequel la section (72) de détermination détermine si l'image observée de manière distincte comprend un nombre prédéterminé ou plus de valeurs de luminance égales ou supérieures au seuil dans une région prédéterminée comparée à l'image de référence et, lorsque l'image observée comprend le nombre prédéterminé ou plus de valeurs de luminance, génère un signal de détermination de brume.

8. Système (1) d'aspiration par ultrasons selon la revendication 5, comprenant en outre une section (81) de détermination d'adhérence qui détermine à partir de l'image observée si un dépôt lié à la diffusion de la brume adhère sur une surface externe d'une partie d'extrémité distale de la section (15) d'observation, dans lequel la section (81) de détermination d'adhérence génère un signal de détermination lorsque la section de détermination d'adhérence détermine que le dépôt a adhéré sur la surface externe.

9. Système (1) d'aspiration par ultrasons selon la revendication 8, dans lequel la section (73) de commande réalise, sur la base de la génération du signal de détermination, une commande pour faire jaillir le fluide depuis une section de jaillissement de fluide (50) prévue au niveau de la partie (47) d'extrémité distale de l'endoscope (14) sur laquelle la section (15) d'observation est prévue vers une surface externe de la partie d'extrémité distale de la section (15) d'observation.

10. Système (1) d'aspiration par ultrasons selon la revendication 9, dans lequel la section (81) de détermination d'adhérence génère le signal de détermination dans le cas d'un résultat de détermination stipulant qu'une différence entre un maximum et un minimum des valeurs de luminance dans l'image observée est égale ou inférieure à une valeur prédéterminée.

11. Système (1) d'aspiration par ultrasons selon la revendication 9, dans lequel la section (81) de détermination d'adhérence comprend une section (81a) d'analyse qui analyse une répartition de fréquence spatiale dans l'image observée, et la section de détermination d'adhérence génère le signal de détermination dans le cas d'un résultat de détermination stipulant que la composante haute fréquence de la fréquence spatiale est égale ou inférieure à une valeur prédéterminée.

12. Système (1) d'aspiration par ultrasons selon la revendication 5, dans lequel la section (73) de commande réalise en outre une commande destinée à faire jaillir un gaz depuis une section (50) de jaillissement de gaz prévue au niveau de la partie d'extrémité distale (47) d'un endoscope (14) sur lequel la section (15) d'observation est prévue vers une surface externe d'une partie d'extrémité distale de la section (15) d'observation au moins pendant une période pendant laquelle le signal d'attaque ultrasonore est généré.

13. Système (1) d'aspiration par ultrasons selon la revendication 12, comprenant en outre
une unité de pneumopéritoine (24) qui délivre et aspire du gaz vers et depuis une cavité de corps, dans laquelle au moins un côté d'extrémité distale de l'endoscope (14) est inséré, dans lequel
la section (73) de commande commande l'opération au moins d'aspiration de gaz dans l'unité pneumopéritoine (24) sur la base d'informations de pression du gaz dans la cavité de corps.

14. Système (1) d'aspiration par ultrasons selon la revendication 2, comprenant en outre :
un dispositif (85a) de chauffage qui est prévu au niveau d'une partie d'extrémité distale (47) d'un endoscope (14) sur lequel la section d'observation (15) est prévue et chauffe un voisinage de la partie d'extrémité distale (47) ; et
une section (85c) d'alimentation qui délivre l'énergie électrique destinée à chauffer le dispositif (85a) de chauffage.

15. Système (1) d'aspiration par ultrasons selon la revendication 10, dans lequel, dans un cas dans lequel le signal de détermination est généré, la section de commande (73) réalise une commande destinée à réduire ou à arrêter l'alimentation en fluide par la section (41) d'alimentation en fluide conjointement avec une commande destinée à arrêter ou à réduire la sortie du signal d'attaque ultrasonore par la section (6) de génération de signal d'attaque ultrasonore.

16. Système (1) d'aspiration par ultrasons selon la revendication 2, comprenant en outre
une ombrelle transparente (92a) prévue de façon à être capable de faire saillie et de se rétracter sur un côté interne d'une gaine enveloppante sur un côté d'extrémité distale de la section (8) d'aspiration par ultrasons, dans laquelle, lorsqu'elle fait saillie, l'ombrelle (92a) couvre une périphérie d'une partie d'extrémité distale d'une section (8) d'aspiration par ultrasons avec une feuille transparente (92e) présentant une forme sensiblement conique.

17. Système (1) d'aspiration par ultrasons selon la revendication 2, comprenant en outre
un sac transparent (95a), dont une extrémité proximale est prévue de manière détachable et dont un côté d'extrémité distale est ouvert, sur un côté d'extrémité distale de la section (8) d'aspiration par ultrasons, dans lequel un bord circonférentiel ouvert du sac (95a) peut être fixé par une pince (96) sur un tissu vivant autour d'une partie d'extrémité distale de la section (8) d'aspiration par ultrasons.

18. Système (1) d'aspiration par ultrasons selon la revendication 2, dans lequel la section (6) de génération de signal d'attaque ultrasonore présente un mode de sortie continue destiné à délivrer en sortie de manière continue le signal d'attaque ultrasonore généré et un mode de sortie par intermittence destiné à délivrer en sortie par intermittence le signal d'attaque ultrasonore à une période prédéterminée incluant une période active et une période inactive.

19. Système (1) d'aspiration par ultrasons selon la revendication 2, dans lequel la section (73) de commande réalise une commande incluant la réduction de la sortie du signal d'attaque ultrasonore en commandant une valeur actuelle du signal d'attaque ultrasonore délivré en sortie par la section de génération de signal d'attaque ultrasonore.
